# EUROPEAN PATENT SPECIFICATION

(11) **EP 1 951 716 B1**
(45) Date of publication and mention of the grant of the patent: **04.05.2011**
(21) Application number: 06837883.5
(22) Date of filing: 16.11.2006
(51) Int. Cl.: C07D 403/14, C07D 417/14, A61K 31/506, A61P 35/00

(54) **AMINOPYRIMIDINES USEFUL AS KINASE INHIBITORS**
ALS KINASEINHIBITOREN GEEIGNETE AMINOPYRIMIDINE
AMINOPYRIMIDINES UTILES EN TANT QU INHIBITEURS DE LA KINASE

(30) Priority: 16.11.2005 US 737064 P
(43) Date of publication of application: 06.08.2008
(73) Proprietor: Vertex Pharmaceuticals, Inc., Cambridge, MA 02139 (US)
(72) Inventor: FRAYSSE, Damien, Oxfordshire OX14 4RY (GB); MILLER, Andrew, Oxfordshire OX14 4RY (GB); ROBINSON, Daniel, Abingdon Oxfordshire Oxon OX14 3GH (GB); PINDER, Joanne, Oxfordshire OX14 4RY (GB)
(74) Representative: Cohausz & Florack
(86) International application number: PCT/US2006/044636
(87) International publication number: WO 2007/059299

(56) References cited:
- WO-A-2005/040159
- US-B2- 6 653 301

## Description

### TECHNICAL FIELD OF THE INVENTION

The present invention relates to compounds useful as inhibitors of kinases. The invention also relates to pharmaceutically acceptable compositions comprising the compounds of the invention, the compounds and compositions for use in the treatment of various disorders, and processes for preparing the compounds.

### BACKGROUND OF THE INVENTION

The search for new therapeutic agents has been greatly aided in recent years by a better understanding of the structure of enzymes and other biomolecules associated with target diseases. One important class of enzymes that has been the subject of extensive study is protein kinases.

Protein kinases are attractive and proven targets for new therapeutic agents to treat a range of human diseases, with examples of kinase inhibitors including Gleevec® and Tarceva®.

Protein kinases constitute a large family of structurally related enzymes that are responsible for the control of a variety of signal transduction processes within the cell. Protein kinases are thought to have evolved from a common ancestral gene due to the conservation of their structure and catalytic function. Almost all kinases contain a similar 250-300 amino acid catalytic domain. The kinases may be categorized into families by the substrates they phosphorylate (e.g., protein-tyrosine, protein-serine/threonine, lipids, etc.). Sequence motifs have been identified that generally correspond to each of these kinase families.

In general, protein kinases mediate intracellular signaling by effecting a phosphoryl transfer from a nucleoside triphosphate to a protein acceptor that is involved in a signaling pathway. These phosphorylation events act as molecular on/off switches that can modulate or regulate the target protein biological function.

These phosphorylation events are ultimately triggered in response to a variety of extracellular and other stimuli. Examples of such stimuli include environmental and chemical stress signals (e.g., osmotic shock, heat shock, ultraviolet radiation, bacterial endotoxin, and H2O2), cytokines (e.g., interleukin-1 (IL-1) and tumor necrosis factor a (TNF-a)), and growth factors (e.g., granulocyte macrophage-colony-stimulating factor (GM-CSF), and fibroblast growth factor (FGF)). An extracellular stimulus may affect one or more cellular responses related to cell growth, migration, differentiation, secretion of hormones, activation of transcription factors, muscle contraction, glucose metabolism, control of protein synthesis, and regulation of the cell cycle.

Many diseases are associated with abnormal cellular responses triggered by protein kinase-mediated events. These diseases include autoimmune diseases, inflammatory diseases, bone diseases, metabolic diseases, neurological and neurodegenerative diseases, cancer, cardiovascular diseases, allergies and asthma, Alzheimer's disease, and hormone-related diseases. Accordingly, there has been a substantial effort in medicinal chemistry to find protein kinase inhibitors that are effective as therapeutic agents. However, considering the lack of currently available treatment options for the majority of the conditions associated with protein kinases, there is still a great need for new therapeutic agents that inhibit these protein targets.

### SUMMARY OF THE INVENTION

This invention provides compounds and pharmaceutically acceptable compositions thereof that are useful as inhibitors of protein kinases. These compounds are represented by formula I: 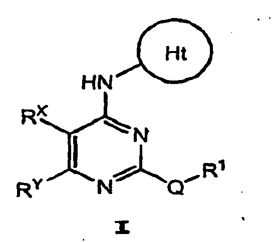 or a pharmaceutically acceptable salt thereof, wherein R¹, R^{X}, R^{Y}, Q, and Ht are as defined herein.

These compounds and pharmaceutically acceptable compositions thereof are useful for inhibiting kinases in vitro, in vivo, and ex vivo. Such uses include treating or preventing a variety of diseases, disorders or conditions, including, but not limited to, autoimmune diseases, inflammatory diseases, bone diseases, metabolic diseases, neurological and neurodegenerative diseases, cancer, cardiovascular diseases, allergies and asthma, Alzheimer's disease, and hormone-related diseases. Other uses include the study of kinases in biological and pathological phenomena; the study of intracellular signal transduction pathways mediated by such kinases; and the comparative evaluation of new kinase inhibitors.

### DETAILED DESCRIPTION OF THE INVENTION

The present invention provides a compound of formula I: 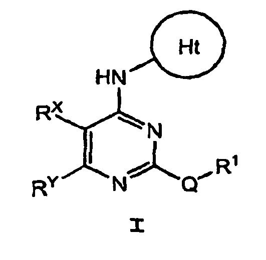 or a pharmaceutically acceptable salt thereof, wherein:
Ht is thiazole or pyrazole, wherein each ring is optionally and independently substituted with R² and R^{2'};
Q is -O-, -NR'-, -S-, -C(R')₂-, or -(C=O)-;
R^{X} is T¹-R³ or L-Z-R³;
R^{Y} is T²-R¹⁰ or L-Z-R¹⁰;
R¹ is T³- (Ring D) ;
Ring D is a 4-7 membered monocyclic ring or 8-10 membered bicyclic ring selected from a heterocyclyl or a carbocyclyl ring, said heterocyclyl ring having 1-4 ring heteroatoms selected from nitrogen, oxygen or sulfur, wherein each substitutable ring carbon of Ring D is independently substituted by oxo, T⁴-R⁵, or V-Z-R⁵, and each substitutable ring nitrogen of Ring D is independently substituted by -R⁴;
each T, T¹, T², T³, and T⁴ is independently a C₁₋₄ alkylidene chain or is absent;
Z is a C₁₋₄alkylidene chain or is absent;
L is -O-**,** -S-, -SO-, -SO₂-, -N(R⁶)SO₂-, -SO₂N(R⁶)-, -N(R⁶)-, -CO-, -CO₂-_{,} -N(R⁶)CO-, -N(R⁶)C(O)O-, -N(R⁶)CON(R⁶)-, -N(R⁶)SO₂N(R⁶)-, -N(R⁶)N(R⁶)-, -C(O)N(R⁶)-, -OC(O)N(R⁶)-, -C(R⁶)₂O-, -C(R⁶)₂S-, -C(R⁶)₂SO-, -C(R⁶)₂SO₂-. -C(R⁶)₂SO₂N(R⁶) -, -C(R⁶)₂N(R⁶)-, -C(R⁶)₂N(R⁶)C(O)-, -C(R⁶)₂N(R⁶)C(O)O-, -C(R⁶)=NN(R⁶)-, -C(R⁶)=N-O- , -C(R⁶)₂N(R⁶)N(R⁶)-, -C(R⁶)₂N(R⁶)SO₂N(R⁶)-, or -C(R⁶)₂N(R⁶)CON(R⁶)-;
R² and R^{2'} are independently -R, -T-W-R⁶, or R⁸, or R² and R^{2'} are taken together with their intervening atoms to form a fused, 5-8 membered, unsaturated or partially unsaturated, ring having 0-3 ring heteroatoms selected from nitrogen, oxygen, or sulfur, wherein each substitutable ring carbon of said fused ring formed by R² and R^{2'} is independently substituted by halo, oxo, -CN, -NO₂, -R⁷, or -V-R⁶, and each substitutable ring nitrogen of said ring formed by R² and R^{2'} is independently substituted by R⁴;
each R³ and R⁵ is independently -R, -halo, -OR, -C(=O)R, -CO₂R, -COCOR, COCH₂COR, -NO₂, -CN, -S(O)R, -S(O)₂R, -SR, -N(R⁴)₂, -CON(R⁷)₂, -SO₂N(R⁷)₂, -OC(=O)R, -N(R⁷)COR, -N(R⁷)CO₂(C₁₋₆ aliphatic), -N(R⁴)N(R⁴)₂, -C=NN(R⁴)₂, -C=N-OR, -N(R⁷)CON(R⁷)₂, -N(R⁷)SO₂N(R⁷)₂, -N(R⁴)SO₂R, or -OC(=O)N(R⁷)₂;
each R is hydrogen, a C₁₋₆ aliphatic group, a C₆₋₁₀ aryl ring, a heteroaryl ring having 5-10 ring atoms, or a heterocyclyl ring having 4-10 ring atoms, the heteroaryl or heterocyclyl ring having 1-4 ring heteroatoms selected from nitrogen, oxygen, or sulfur, the aliphatic group and each R ring being optionally substituted by R⁹;
each R⁴ is -R⁷, -COR⁷, -CO₂ (optionally substituted C₁₋₆ aliphatic), -CON(R⁷)₂, or -SO₂R⁷;
V is -O-, -S-, -SO-, -SO₂-. -N(R⁶)SO₂-, -SO₂N(R⁶)-, -N(R⁶)-, -CO-, -CO₂-, -N(R⁶)CO-, -N(R⁶)C(O)O-, -N(R⁶)CON(R⁶)-, -N(R⁶)SO₂N(R⁶)-, -N(R⁶)N(R⁶)-, -C(O)N(R⁶)-, -OC(O)N(R⁶)-, -C(R⁶)₂O-, -C(R⁶)₂S-, -C(R⁶)₂SO-, -C(R⁶)₂SO₂-, -C(R⁶)₂SO₂N(R⁶)-, C(R⁶)₂N(R⁶)-, -C(R⁶)₂N(R⁶)C(O)-, -C(R⁶)₂N(R⁶)C(O)O-, C(R⁶)=NN(R⁶)-, -C(R⁶)=N-O-_{.} -C(R⁶)₂N(R⁶)N(R⁶)-, -C(R⁶)₂N(R⁶)SO₂N(R⁶)-, or -C(R⁶)₂N(R⁶)CON(R⁶)-;
W is -C(R⁶)₂O-, -C(R⁶)₂S-, -C(R⁶)₂SO-, -C(R⁶)₂SO₂-, -C(R⁶)2SO₂N(R⁶)-, -C(R⁶)₂N(R⁶)-, -CO-, -CO₂-, -C(R⁶)OC(O)-, -C(R⁶)OC(O)N(R⁶)-, -C(R⁶)₂N(R⁶)CO-, -C(R⁶)₂N(R⁶)C(O)O-, -C(R⁶)=NN(R⁶)-, -C(R⁶) =N-O-, -C(R₆)₂N(R⁶)N(R⁶)-, -C(R⁶)₂N(R⁶)SO₂N(R⁶)-, -C(R⁶)₂N(R⁶)CON(R⁶)-, or -CON(R⁶)-;
each R⁶ is independently hydrogen or an optionally substituted C₁₋₆ aliphatic group, or two R⁶ groups on the same nitrogen atom are taken together with the nitrogen atom to form an optionally substituted 4-6 membered heterocyclyl or heteroaryl ring; and
each R⁷ is independently hydrogen or an optionally substituted C₁₋₆ aliphatic group, or two R⁷ on the same nitrogen are taken together with the nitrogen to form an optionally substituted 5-8 membered heterocyclyl or heteroaryl ring;
each R⁸ is halogen, -CN, or -NO₂;
each R⁹ is -R', -halo, -OR', -C(=O)R', -CO₂R', -COCOR', COCH₂COR' , -NO₂, -CN, -S(O)R' , -S(O)₂R' , -SR', -N(R')₂, -CON (R')₂, -SO₂N (R')₂, -OC(=O)R', -N(R')COR', -N(R')CO₂(C₁₋₆ -CON(R')₂, -SO₂N(R')₂, -OC(=O)R', -N(R')COR', -N(R')CO₂(C₁₋₆ aliphatic), -N(R')N(R')₂, -C=NN(R')₂, -C=N-OR', -N(R')CON(R')₂, -N(R')SO₂N(R')₂, -N(R')SO₂R', or -OC(=O)N(R')₂;
each R¹⁰ is a 4-membered heterocyclic ring containing 1-2 heteroatoms selected from O, NR¹¹, and S; each R¹⁰ is optionally substituted with 0-3 occurrences of J;
each J is independently -halo, -OR, oxo, C₁₋₆ aliphatic, -C(=O)R, -CO₂R, -COCOR, COCH₂COR, -NO₂, -CN, -S(O)R, -S(O)₂R, -SR, -N(R⁴)₂, -CON(R⁷)2, -SO₂N(R⁷)₂, -OC(=O)R, -N(R⁷)COR, -N(R⁷)CO₂(C₁₋₆ aliphatic), -N(R⁴)N(R⁴)₂, =NN(R⁴)₂, =N-OR, -N(R⁷)CON(R⁷)₂, -N(R⁷)SO₂N(R⁷)₂, -N(R⁴)SO₂R, or -OC(=O)N(R⁷)₂; or
2 J groups, on the same atom or on different atoms, together with the atom(s) to which they are bound, form a 3-8 membered saturated, partially saturated, or unsaturated ring having 0-2 heteroatoms selected from O, N, or S;
each R¹¹ is -R⁷, -COR⁷, -CO₂(optionally substituted C₁₋₆ aliphatic), -CON(R⁷)₂, or -SO₂R⁷;
each R' is independently hydrogen or a C₁₋₆aliphatic group optionally substituted with 0-4 occurrences of NH₂, NH(C₁₋₄aliphatic), N(C₃₋₄aliphatic)₂, halogen, C₁₋₄aliphatic, OH, O(C₁₋₄aliphatic), NO₂, CN, CO₂H, CO₂(C₁₋₄aliphatic), O(haloC₁₋₄ aliphatic), or haloC₁₋₄aliphatic; or, two R', together with the atom(s) to which they are attached, form an optionally substituted 3-6 membered carbocyclyl or heterocyclyl.

In some embodiments, the present invention provides a compound of formula I: 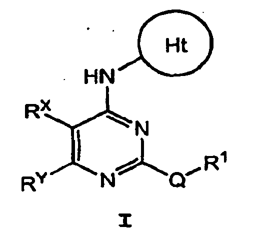 or a pharmaceutically acceptable salt thereof, wherein:
Ht is thiazole or pyrazole; Ht is optionally and independently substituted with R² and R^{2'};
Q is -O-, -NR' -, -S-, -C(R')₂-, or -(C=O)-;
R^{X} is H, C₁₋₆aliphatic, NO₂, CN, halo, NH₂, N(C₁₋₄aliphatic), N(C₁₋₄aliphatic)₂, O(C₁₋₄aliphatic), OH, or -N(C=O)(C₁₋₄aliphatic); wherein said aliphatic is optionally substituted with 1-3 fluoro;
R^{Y} is -Z-R¹⁰;
R¹ is T-(Ring D);
Ring D is a 4-7 membered monocyclic heterocyclyl or a carbocyclyl ring, wherein said heterocyclyl has 1-4 ring heteroatoms selected from O, N, and S; Ring D is optionally fused to Ring D';
Ring D' is a 5-8 membered partially saturated or fully unsaturated monocyclic ring containing 0-4 ring heteroatoms selected from nitrogen, oxygen or sulfur;
Ring D and Ring D' are each independently and optionally substituted with 0-4 occurrences of oxo or -Z-R⁵;
each T is independently a C₁₋₄ alkylidene chain or is absent;
R² and R^{2'} are independently -R, -W-R⁶, or R⁸; or R² and R^{2'} are taken together with their intervening atoms to form a fused, 5-8 membered, unsaturated or partially unsaturated, ring having 0-3 ring heteroatoms selected from nitrogen, oxygen, or sulfur; wherein the 5-8 membered ring is independently substituted with halo, oxo, -CN, -NO₂, -R⁷, or -Y-R⁶;
each Z, Y, and W is independently a bond or a C₁₋₁₀ alkylidene chain wherein up to six methylene units of the alkylidene chain are optionally replaced by V;
each V is selected from -O-, -C(=O)-, -S(O)-, -S(O)₂-, -S-, or -N(R⁴)-;
each R³ and R⁵ is independently -R, -halo, -OR, -C(=O)R, -CO_{2R}, -COCOR, COCH₂COR, -NO₂, -CN, -S(O)R, -S(O)₂R, -SR, -N(R⁴)₂, -CON(R⁷)₂, -SO₂N(R⁷)₂, -OC(=O)R, -N(R⁷)COR, -N(R⁷)CO₂(C₁₋₆ aliphatic), -N(R⁴)N(R⁴)₂, -C=NN(R⁴)₂, -C=N-OR, -N(R⁷)CON(R⁷)₂, -N(R⁷)SO₂N(R⁷)₂, -N(R⁴)SO₂R, or -OC(=O)N(R⁷)₂;
each R is hydrogen, a C₁₋₆ aliphatic group, a C₆₋₁₀aryl ring, a heteroaryl ring having 5-10 ring atoms, or a heterocyclyl ring having 4-10 ring atoms; wherein said heteroaryl or heterocyclyl ring has 1-4 ring heteroatoms selected from nitrogen, oxygen, or sulfur; R is optionally substituted with 0-6 R⁹;
each R⁴ is -R⁷, -COR⁷, -CO₂R⁷, -CON(R⁷)₂, or -SO₂R⁷;
each R⁶ is independently hydrogen or an optionally substituted C₁₋₆ aliphatic group; or two R⁶ groups on the same nitrogen atom are taken together with the nitrogen atom to form an optionally substituted 4-6 membered heterocyclyl or heteroaryl ring;
each R⁷ is independently hydrogen or an optionally substituted C₁₋₆ aliphatic group; or two R⁷ on the same nitrogen are taken together with the nitrogen to form an optionally substituted 4-8 membered heterocyclyl or heteroaryl ring containing 1-4 heteroatoms selected from nitrogen, oxygen, or sulfur;
each R⁸ is halogen, -CN, or -NO₂;
each R⁹ is -R', -halo, -OR', -C(=O)R', -CO₂R', -COCOR', COCH₂COR', -NO₂, -CN, -S(O)R', -S(O)₂R', -SR', -N(R')₂, -CON(R')₂, -SO₂N(R')₂, -OC(=O)R', -N(R')COR', -N(R')CO₂(C₁₋₆ aliphatic), -N(R')N(R')₂, -N(R')CON(R')₂, -N(R')SO₂N(R')₂, -N(R')SO₂R', -OC(=O)N(R')₂, =NN(R')₂, =N-OR', or =O;
each R¹⁰ is a 4-membered heterocyclic ring containing 1 heteroatom selected from O, NR¹¹, and S; each R¹⁰ is optionally substituted with 0-6 occurrences of J;
each J is independently R, -halo, -OR, oxo, -C(=O)R, -CO₂R, -COCOR, -COCH₂COR, -NO₂, -CN, -S(O)R, -S(O)₂R, -SR, -N(R⁴)₂, -CON(R⁷)₂, -SO₂N(R⁷)2, -OC(=O)R, -N(R⁷COR, -N(R⁷)CO₂(C₁₋₆ aliphatic), -N(R⁴)N(R⁴)₂, =NN(R⁴)₂, =N-OR, -N(R⁷)CON(R⁷)₂, -N(R⁷)SO₂N(R⁷)₂, -N(R⁴)SO₂R, -OC(=O)N(R⁷)₂, or -OP(=O)(OR")₂; or
2 J groups, on the same atom or on different atoms, together with the atom(s) to which they are bound, form a 3-8 membered saturated, partially saturated, or unsaturated ring having 0-2 heteroatoms selected from O, N, or S; wherein 1-4 hydrogen atoms on the ring formed by the 2 J groups is optionally replaced with halo, C₁₋₃alkyl, or -O(C₁₋₃alkyl); or two hydrogen atoms on the ring are optionally replaced with oxo or a spiro-attached C₃₋₄ cycloalkyl; wherein said C₁₋₃alkyl is optionally substituted with 1-3 fluorine;
each R¹¹ is -R⁷, -COR⁷ -CO₂R⁷, -CON(R⁷)₂, or - SO₂R₇;
each R' is independently hydrogen or a C₁₋₆ aliphatic group optionally substituted with 0-4 occurrences of NH₂, NH(C₁₋₄aliphatic), N(C₁₋₄aliphatic)₂, halogen, C₁₋₄aliphatic, OH, O(C₁₋₄aliphatic), NO₂, CN, CO₂H, CO₂(C₁₋₄aliphatic), O(haloC₁₋₄ aliphatic), or haloC₁₋₄aliphatic; or, two R', together with the atom(s) to which they are attached, form an optionally substituted 3-6 membered carbocyclyl or heterocyclyl; and
each R" is independently H or C₁₋₂alkyl.

In some embodiments, the present invention provides a compound of formula I: 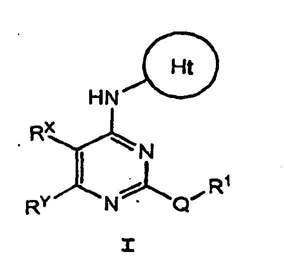 or a pharmaceutically acceptable salt thereof, wherein:
Ht is thiazole or pyrazole, wherein each ring is optionally and independently substituted with R² and R^{2'};
Q is -O-, -NR'-, -S-, or -C(R')₂-;
R^{X} is H, C₁₋₆aliphatic, NO₂, CN, halo, NH₂, N(C₁₋₄aliphatic), N(C₁₋₄aliphatic)₂, O(C₁₋₄aliphatic), OH, or -N(C=O)(C₁₋₄aliphatic); wherein said aliphatic is optionally substituted with 1-3 fluoro;
R^{Y} is T²-R¹⁰ or L-Z-R¹⁰;
R¹ is T³- (Ring D) ;
Ring D is a 4-7 membered monocyclic heterocyclyl or a carbocyclyl ring, wherein said heterocyclyl has 1-4 ring heteroatoms selected from O, N, and S; Ring D is optionally fused to Ring D';
Ring D' is a 5-8 aromatic, partially saturated, or fully unsaturated ring containing 0-4 ring heteroatoms selected from nitrogen, oxygen or sulfur;
Ring D and Ring D' are each independently optionally substituted with 0-4 occurrences of oxo, T⁴-R⁵, or V-Z-R⁵;
each T, T³, and T⁴ is independently a C₁₋₄ alkylidene chain or is absent;
Z is a C₁₋₄ alkylidene chain or is absent;
L is -O-, -S-, -SO-, -SO₂-, -N(R⁶)SO₂-, -SO₂N(R⁶)-, -N(R⁶)-, -CO-, -CO₂-, -N(R⁶)CO-, -N(R⁶)C(O)O-, -N(R⁶)CON(R⁶)-, -N(R⁶)SO₂N(R⁶)-, -N(R⁶)N(R⁶)-, -C(O)N(R⁶)-, -OC(O)N(R⁶)-, -C(R⁶)₂O-. -C(R⁶)₂S-, -C(R⁶)₂SO-, -C(R⁶)₂SO₂-, -C(R⁶)₂SO₂N(R⁶)-, -C(R⁶)₂N(R⁶)-, -C(R⁶)₂N(R⁶)C(O)-, -C(R⁶)₂N(R⁶)C(O)O-, -C(R⁶)=NN(R⁶)-, -C(R⁶)=N-O-, -C(R⁶)2N(R⁶)N(R⁶)-, -C(R⁶)₂N(R⁶)SO₂N(R⁶)-, or -C(R⁶)₂N(R⁶)CON(R⁶)-;
T² is independently absent or a C₁₋₁₀ alkylidene chain wherein up to six C units of the alkylidene chain are optionally replaced by -O-, -C(=O)-, -S(O)-, -S(O)₂-, -S-, or -N(R⁴)-; T² is optionally substituted with 0-6 J^{T} groups;
R² and R^{2'} are independently -R, -T-W-R⁶, or R⁸, or R² and R^{2'} are taken together with their intervening atoms to form a fused, 5-8 membered, unsaturated or partially unsaturated, ring having 0-3 ring heteroatoms selected from nitrogen, oxygen, or sulfur, wherein each substitutable ring carbon of said fused ring formed by R² and R^{2'} is independently substituted by halo, oxo, -CN, -NO₂, -R⁷, or -V-R⁶, and each substitutable ring nitrogen of said ring formed by R² and R^{2'} is independently substituted by R⁴;
R⁵ is -R, -halo, -OR, -C(=O)R, -CO₂R, -COCOR, COCH₂COR, -NO₂, -CN, -S(O)R, -S(O)₂R, -SR, -N(R⁴)₂, -CON(R⁷)₂, -SO₂N(R⁷)₂, -OC(=O)R, -N(R⁷)COR, -N(R⁷)CO₂(C₁₋₆ aliphatic), -N(R⁴)N(R⁴)₂, -C=NN(R⁴ )₂, -C=N-OR, -N(R⁷)CON(R⁷)₂, -N(R⁷)SO₂N(R⁷)₂, -N(R⁴)SO₂R, Or - OC(=O)N(R⁷)₂;
each R is hydrogen, a C₁₋₁₀ aliphatic group, a C₆₋₁₀ aryl ring, a heteroaryl ring having 5-10 ring atoms, or a heterocyclyl ring having 4-10 ring atoms, the heteroaryl or heterocyclyl ring having 1-4 ring heteroatoms selected from nitrogen, oxygen, or sulfur, the aliphatic group and each R being optionally substituted by 0-6 R⁹;
each R⁴ is -R⁷, -COR⁷, -CO₂R⁷, -CON(R⁷)₂, or -SO₂R⁷;
V is -O-, -S-, -SO-, -SO₂-, -N(R⁶)SO₂-, -SO₂N(R⁶)-, -N(R⁶)-, -CO-, -CO₂-, -N(R⁶)CO-, -N(R⁶)C(O)O-, -N(R⁶)CON(R⁶)-, -N(R⁶)SO₂N(R⁶)-, -N(R⁶)N(R⁶)-, -C(O)N(R⁶)-, -OC(O)N(R⁶)-, -C(R⁶)₂O-, -C(R⁶)₂S-, -C(R⁶)₂SO-, -C(R⁶)2SO₂-, -C(R⁶)₂SO₂N(R⁶)-, C(R⁶)₂N(R⁶)-, -C(R⁶)₂N(R⁶)C(O)-, -C(R⁶)₂N(R⁶)C(O)O-, C(R⁶)=NN(R⁶)-, -C(R⁶)=N-O-, -C(R⁶)₂N(R⁶)N(R⁶)-, -C(R⁶)₂N(R⁶)SO₂N(R⁶)-, or -C(R⁶)₂N(R⁶)CON(R⁶)-;
W is -C(R⁶)₂O-, -C(R⁶)₂S-, -C(R⁶)₂SO-, -C(R⁶)₂SO₂-, -C(R⁶)₂SO₂N(R⁶)-, -C(R⁶)₂N(R⁶)-, -CO-, -CO₂-, -C(R⁶)₂OC(O)-, -C(R⁶)₂OC(O)N(R⁶)-, -C(R⁶)₂N(R⁶)CO-, -C(R⁶)₂N(R⁶)C(O)O-, -C(R⁶)=NN(R⁵)-, -C(R⁶)=N-O-, -C(R⁶)2N(R⁶)N(R⁶)-_{.} -C(R⁶)₂N(R⁶)SO₂N(R⁶)-, -C(R⁶)₂N(R⁶)CON(R⁶)-, or -CON(R⁶)-;
each R⁶ is independently hydrogen or C₁₋₆ aliphatic optionally substituted with 0-3 J⁶; or two R⁶ groups on the same nitrogen atom are taken together with the nitrogen atom to form a 4-8 membered heterocyclyl or heteroaryl ring; wherein said heterocyclyl or heteroaryl ring is optionally substituted with 0-4. J⁶;
each R⁷ is independently hydrogen; C₁₋₆ aliphatic; a 5-membered heteroaryl containing 0-4 heteroatoms selected from O, N, or S; or phenyl; each R⁷ is optionally substituted with 0-3 J⁷; or two R⁷ on the same nitrogen are taken together with the nitrogen to form an optionally substituted 4-8 membered heterocyclyl or heteroaryl ring; wherein said heterocyclyl or heteroaryl ring is optionally substituted with 0-4 J⁷;
each R⁸ is halogen, -CN, or -NO₂;
each R⁹ is -R', -halo, -OR' , -C(=O)R', -CO₂R', -COCOR', COCH₂COR' , -NO₂, -CN, -S(O)R', -S(O)₂R', -SR' , -N(R')₂, -CON(R')₂, -SO₂N(R')₂, -OC(=O)R', -N(R')COR', -N(R')CO₂(C₁₋₆ aliphatic), -N(R')N(R')₂, -N(R')CON(R')₂, -N(R')SO₂N(R')₂, -N(R')SO₂R', -OC(=O)N(R')₂, =NN(R')₂, =N-OR', =NR', or =O;
each R¹⁰ is a 4-membered heterocyclic ring containing 1 heteroatom selected from O, NR¹¹, and S; each R¹⁰ is optionally substituted with 0-6 occurrences of J;
each J and J^{T} is independently R, -halo, -OR, -C(=O)R, -CO₂R, -COCOR, COCH₂COR, -NO₂, -CN, -S(O)R, -S(O)₂R, -SR, -N(R⁴)₂, -CON(R⁷)₂, -SO₂N(R⁷)₂, -OC(=O)R, -N(R⁷)COR, -N(R⁷)CO₂(C₁₋₆ aliphatic), -N(R⁴)N(R⁴)₂, =NN(R⁴)₂, =N-OR, =NR', =O, -N(R⁷)CON(R⁷)₂, -N(R⁷)SO₂N(R⁷)₂, -N(R⁴)SO₂R, -OC(=O)N(R⁷)₂, or -OP(=O)(OR")₂; or
each J⁶ and J⁷ is independently NH₂, NH(C₁₋₄aliphatic), N(C₁₋₄aliphatic)₂, halogen, C₁₋₄aliphatic, OH, O(C₁₋₄aliphatic), NO₂, CN, CO₂H, CO₂(C₁₋₄aliphatic), O(haloC₁₋₄aliphatic), or haloC₁₋₄aliphatic;
2 J or J^{T} groups, on the same atom or on different atoms, together with the atom(s) to which each set of J for J^{T} atoms are bound, form a 3-8 membered saturated, partially saturated, or unsaturated ring having 0-2 heteroatoms selected from O, N, or S; wherein 1-4 hydrogen atoms on the ring formed by the 2 J or J^{T} groups is optionally replaced with halo, C₁₋₃alkyl, or -O(C₁₋₃alkyl); wherein said C₁₋₃alkyl is optionally substituted with 1-3 fluorine; or
two hydrogen atoms on the same atom in the ring formed by the 2 J or J^{T} groups are optionally replaced with oxo;
each R¹¹ is -R⁷, -COR⁷, -CO₂R⁷, -CON(R⁷)₂, or -SO₂R⁷;
each R' is independently hydrogen or a C₁₋₆ aliphatic group optionally substituted with 0-4 occurrences of NH₂, NH(C₁₋₄aliphatic), N(C₁₋₄aliphatic)₂, halogen, C₁₋₄aliphatic, OH, O(C₁₋₄aliphatic), NO₂, CN, CO₂H, CO₂(C₁₋₄aliphatic), CONH₂, CONH(C₁₋₄aliphatic), CON(C₁₋₄aliphatic)₂, O(haloC₁₋₄ aliphatic), or haloC₁₋₄aliphatic; or, two R', together with the atom(s) to which they are attached, form =O, an optionally substituted 3-6 membered carbocyclyl, or heterocyclyl;
each R" is independently H or C₁₋₂alkyl.

In some embodiments, Ht is 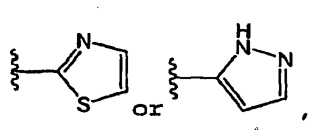 wherein each ring is optionally and independently substituted with R² and R^{2'};

In some embodiments, Q is -C(=O)-. In other embodiments, Q is a heteroatom selected from -O-, -NR'- or -S-. In some embodiments, Q is -NR'- or -S-. In some embodiments, Q is -NR'- or -O-. In some embodiments, Q is -S-. In other embodiments is -O-. In yet other embodiments, Q is -NR'-.

In some embodiments, R¹ is T-(Ring D). In some embodiments, T is absent.

In some embodiments, Ring D is an optionally substituted 4-7 membered monocyclic ring selected from a heterocyclyl or a carbocyclyl ring. In some embodiments, said heterocyclyl ring has 1-4 ring heteroatoms selected from nitrogen, oxygen or sulphur. In some embodiments, said heterocyclyl ring has 1-2 heteroatoms selected from O, N, and S. In some embodiments Ring D is an optionally substituted 4-7 membered carbocyclyl ring. In some embodiments Ring D is an optionally substituted 4-7 membered heterocyclic ring, wherein said heterocyclic ring has 1-2 heteroatoms selected from O, N, and S. In some embodiments, said heterocyclyl ring is piperidinyl, pyrrolidinyl, or morpholinyl. In one embodiment said heterocyclyl is piperidinyl. In another embodiment, said heterocyclyl is pyrrolidinyl.

In some embodiments, Ring D' is phenyl.

As would be understood by a skilled practitioner, when two rings are fused, the two rings share two adjacent atoms and also the bond or bonds between the adjacent atoms. For example, piperidine fused with phenyl could form tetrahydroisoquinoline.

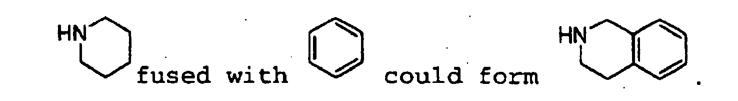

phenyl fused with pyrimidine could form quinazoline.

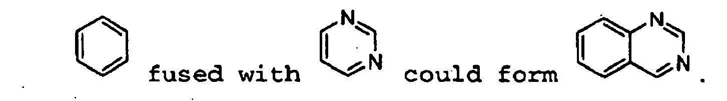

Phenyl fused with pyrrolidine could form indoline.

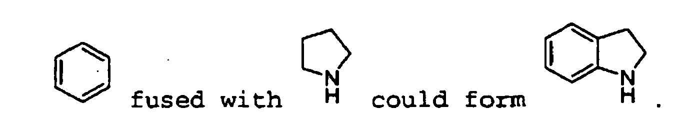

The fused ring can be rotated in any chemically stable orientation. For example, a phenyl fused with an imidazole could form one of three possible compounds:

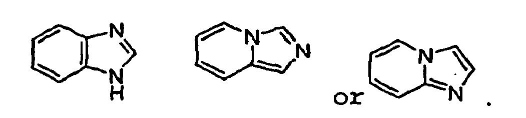

In some embodiments; Ring D is mono-substituted in the 4-position with T⁴-R⁵ or V-Z-R⁵-. In some embodiments, Ring D is optionally substituted in the 4-position with V-Z-R⁵.

In some embodiments, V is -N(R⁶)CO-, -C(O)N(R⁶)-, =O-, -N(R⁶)-, or -N(R⁶)SO₂-. In other embodiments, V is -N(R⁶)CO- or -C(O)N(R⁶)-_{.}

In some embodiments, Ring D is substituted with -V-Z-R⁵. In some embodiments, V is -CO- or -CO₂-. In some embodiments z is absent. In some embodiments, R⁵ is R. In some embodiments, R⁵ is C₁₋₆ aliphatic or phenyl optionally substituted with halo. In some embodiments, Ring D is substituted with -CO₂(C₁₋₆aliphatic) or -CO(phenyl).

Unless otherwise indicated, the term "absent," when referring to a substituent linked to two other substituents, is synonymous with the term "a bond." In both cases, the variable is defined as the bond that links together the other two substituents to which it is bound. For example, if the substituent is -V-Z-R⁵; and Z is "a bond" or Z "is absent; " then the substituent becomes -V-R⁵. z becomes the bond that joins V and R⁵.

In other embodiments, Ring D is substituted with -Z-R⁵.

In some embodiments, Z is a C₁₋₁₀ alkylidene chain wherein up to six methylene units of the alkylidene chain are optionally replaced by -O-, -C(=O)-, -S(O)-, -S(O)₂-, -S-, or -N(R⁶)-. In some embodiments, the methylene units of Z are replaced by -O-, -S-, -SO-, -SO₂-, -N(R⁶)SO₂-, -SO₂N(R⁶)-, -N(R⁶)-, -CO-, -CO₂-, -N(R⁶)CO-, -N(R⁶)C(O)O-, -N(R⁶)CON(R⁶)-, -N(R⁶)SO₂N(R⁶)-, -N(R⁶)N(R⁶)-, -C(O)N(R⁶)-, -OC(O)N(R⁶)-, -C(R⁶)₂O-, -C(R⁶)₂S-, -C(R⁶)₂SO-, -C(R⁶)₂SO₂-, -C(R⁶)₂SO₂N(R⁶)-, -C(R⁶)₂N(R⁶)-, -C(R⁶)₂N(R⁶)C(O)-, -C(R⁶)₂N(R⁶)C(O)O-, -C(R⁶)=NN(R⁶)-, -C(R⁶)=N-O-, -C(R⁶)₂N(R⁶)N(R⁶)-, -C(R⁶)₂N(R⁶)SO₂N(R⁶)-, or -C(R⁶)₂N(R⁶)CON(R⁶)-.

In other embodiments, Z is absent.

In some embodiments, T³ is absent.

In other embodiments, T³ is a C₁₋₄ alkylidene chain.

In certain embodiments, the substituents in R⁶ and R⁷ are independently selected from R⁹.

In another embodiment, the optionally substituted aliphatic group of R⁶ is a C₁₋₄ aliphatic group.

In some embodiments, R⁷ is an optionally substituted group selected from C₁₋₆ aliphatic, C₆₋₁₀ aryl, or a heteroaryl ring having 5-10 ring atoms. In some embodiments, R⁷ is an optionally substituted group selected from C₁₋₆ aliphatic or phenyl. In some embodiments, said phenyl group is optionally substituted with halo.

In another embodiment R² is H or C₁₋₆ aliphatic (which is unsubstituted in certain embodiments).

In another embodiment R² is H or C₁₋₃ aliphatic (which is unsubstituted in certain embodiments).

In another embodiment R^{2'} is H or C₁₋₃ aliphatic (which is unsubstituted in certain embodiments). In another embodiment R^{2'} is H.

In some embodiments, R² is C₁₋₆ aliphatic and R^{2'} is H.

In one embodiment, R^{x} is -R, halogen, -NO₂, -CN, -CO₂R, -OR, or -SR. In another embodiment, R^{x} is H or F.

In one embodiment, R^{Y} is T²-R¹⁰. In some embodiments, T² is absent. In another embodiment, R^{Y} is -Z-R¹⁰. In some embodiments, Z is absent. In other embodiments, Z is a C₁₋₆ alkylidene chain wherein 1-2 methylene units of Z is optionally replaced by O, -N(R⁶)-, or S. In yet other embodiments, Z is a C₁₋₄ alkylidene chain.

In other embodiments, R¹⁰ is a 4-membered heterocyclic ring containing 1 heteroatom. In some embodiments, R¹⁰ is azetidine. In other embodiments, R¹⁰ is

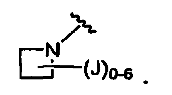

In some embodiments, R^{Y} is represented by formula i:

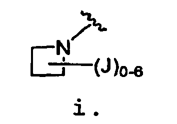

In some embodiments, J is C₁₋₄alkyl, C₃₋₆alkyl O(C-₁₋₃₄alkyl), OH, CN, or F. In other embodiments, J is CH₃, OCH₃, O(CH₂CH₃), OCH (CH₃)₂, OC (CH₃)₃, OH, CN, or F. In yet other embodiments, J is F or cyclopropyl.

In another embodiment, R^{Y} is L-Z-R¹⁰. In some embodiments, L is -O-, -N(R⁶)-, or -S-. In some embodiments, Z is a C₁₋₄ alkylidene chain. In other embodiments, Z is absent. In some embodiments, R^{Y} is represented by formula ii-a or ii-a' :

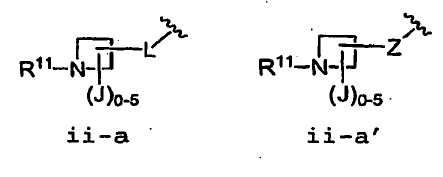

In other embodiments, R^{Y} is represented by formula ii-b or ii-b' :

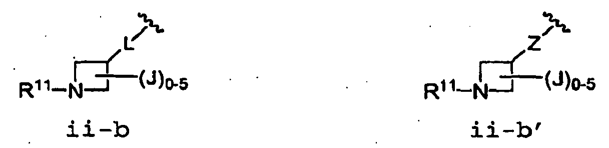

In some embodiments, R¹¹ is H. In other embodiments, R¹¹ is an optionally substituted C₁₋₆ aliphatic group. In yet other embodiments, R¹¹ is -COR⁷, -CO₂R⁷, -CON(R⁷)₂, or -SO₂R⁷.

In one embodiment, a compound of this invention is represented by formula Ia : 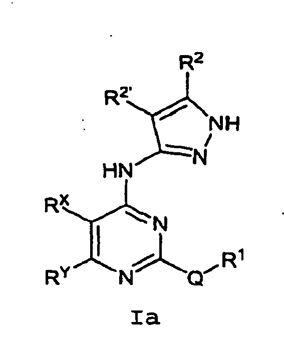 wherein the variables are as defined in any embodiment herein. In some embodiments, Q is S or O. In some embodiments, Q is S. In other embodiments, Q is O.

In another embodiment, a compound of this invention is represented by formula Ib: 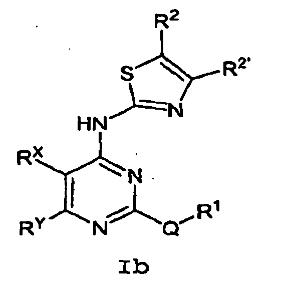 wherein the variables are as defined in any embodiment herein.

In some embodiments, Q is S or O. In some embodiments, Q is S. In other embodiments, Q is O.

In some embodiments, R^{2'} is H or optionally substituted C₁₋₃ aliphatic. In other embodiments, R^{2'} is H. In yet other embodiments, R² is H or optionally substituted C₁₋₃ aliphatic.

In another embodiment, R⁵ is an optionally substituted group selected from C₁₋₆ aliphatic or phenyl.

As described above, the variables of formula Ia and Ib are as defined in any embodiment herein. For the avoidance of doubt, these embodiments include, but are not limited to, compounds wherein R^{y} is -Z-R¹⁰; wherein if Z is absent, R¹⁰ is a 4-membered heterocyclic ring containing 1 heteroatom selected from O, NR¹¹, and S. It should be understood that when the heterocyclic ring is attached via a nitrogen atom (as shown below in formula i), R¹¹ is absent.

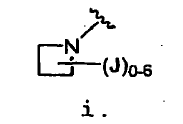

In another embodiment, this invention includes a compound selected from Table 1 (or a pharmaceutically acceptable salt thereof):

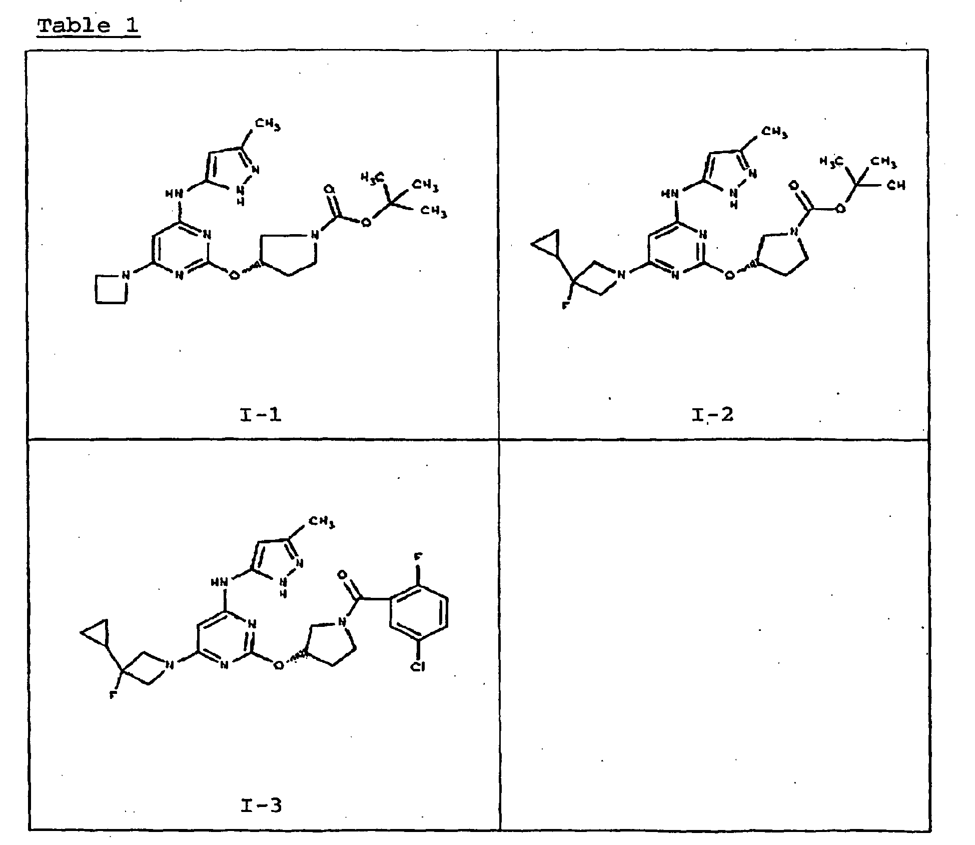

For purposes of this invention, the chemical elements are identified in accordance with the Periodic Table of the Elements, CAS version, Handbook of Chemistry and Physics, 75th Ed. Additionally, general principles of organic chemistry are described in texts known to those of ordinary skill in the art, including, for example, "Organic Chemistry", Thomas Sorrell, University Science Books, Sausalito: 1999, and "March's Advanced Organic Chemistry", 5th Ed., Ed.: Smith, M.B. and March, J., John Wiley & Sons, New York: 2001.

As described herein, a specified number range of atoms includes any integer therein. For example, a group having from 1-4 atoms could have 1, 2, 3, or 4 atoms.

As described herein, compounds of the invention may optionally be substituted with one or more substituents, such as are illustrated generally above, or as exemplified by particular classes, subclasses, and species of the invention. It will be appreciated that the phrase "optionally substituted" is used interchangeably with the phrase "substituted or unsubstituted." In general, the term "substituted", whether preceded by the term "optionally" or not, refers to the replacement of hydrogen radicals in a given structure with the radical of a specified substituent. Unless otherwise indicated, an optionally substituted group may have a substituent at each substitutable position of the group, and when more than one position in any given structure may be substituted with more than one substituent selected from a specified group, the substituent may be either the same or different at every position. Combinations of substituents envisioned by this invention are preferably those that result in the formation of stable or chemically feasible compounds.

The term "stable", as used herein, refers to compounds that are not substantially altered when subjected to conditions to allow for their production, detection, and preferably their recovery, purification, and use for one or more of the purposes disclosed herein. In some embodiments, a stable compound or chemically feasible compound is one that is not substantially altered when kept at a temperature of 40 °C or less, in the absence of moisture or other chemically reactive conditions, for at least a week.

The term "aliphatic" or "aliphatic group", and the like, as used herein, means an unbranched or branched, straight-chain or cyclic, substituted or unsubstituted hydrocarbon that is completely saturated or that contains one or more units of unsaturation that has a single point of attachment to the rest of the molecule. Unless otherwise specified, aliphatic groups contain 1-20 aliphatic carbon atoms. In some embodiments, aliphatic groups contain 1-10 aliphatic carbon atoms. In other embodiments, aliphatic groups contain 1-8 aliphatic carbon atoms. In still other embodiments, aliphatic groups contain 1-6 aliphatic carbon atoms, and in yet other embodiments aliphatic groups contain 1-4 aliphatic carbon atoms. Suitable aliphatic groups include, but are not limited to, linear or branched, substituted or unsubstituted alkyl, alkenyl, or alkynyl groups. Specific examples include, but are not limited to, methyl, ethyl, isopropyl, n-propyl, sec-butyl, vinyl, n-butenyl, ethynyl, and tert-butyl.

The term "cycloaliphatic" (or "carbocycle" or "carbocyclyl" or "cycloalkyl" and the like) refers to a monocyclic C₃-C₈ hydrocarbon or bicyclic C₈-C₁₂ hydrocarbon that is completely saturated or that contains one or more units of unsaturation, but which is not aromatic, that has a single point of attachment to the rest of the molecule wherein any individual ring in said bicyclic ring system has 3-7 members. Suitable cycloaliphatic groups include, but are not limited to, cycloalkyl and cycloalkenyl groups. Specific examples include, but are not limited to, cyclohexyl, cyclopropenyl, and cyclobutyl.

In the compounds of this invention, rings include linearly-fused, bridged, or spirocyclic rings. Examples of bridged cycloaliphatic groups include, but are not limited to, bicyclo[3.3.2]decane, bicyclo[3.1.1]heptane, and bicyclo[3.2.2]nonane.

The term "heterocycle", "heterocyclyl", "heterocycloaliphatic", or "heterocyclic", and the like, as used herein means non-aromatic, monocyclic, bicyclic, or tricyclic ring systems in which one or more ring members are an independently selected heteroatom. In some embodiments, the "heterocycle", "heterocyclyl", "heterocycloaliphatic", or "heterocyclic" group has three to fourteen ring members in which one or more ring members is a heteroatom independently selected from oxygen, sulfur, nitrogen, or phosphorus, and each ring in the system contains 3 to 7 ring members. Examples of bridged heterocycles include, but are not limited to, 7-aza-bicyclo[2.2.1]heptane and 3-aza-bicyclo[3.2.2]nonane.

Suitable heterocycles include, but are not limited to, 3-1H-benzimidazol-2-one, 3-(1-alkyl)-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-tetrahydropiperazinyl, 2-tetrahydropiperazinyl, 3-tetrahydropiperazinyl, 1-piperidinyl, 2-piperidinyl, 3-piperidinyl, 1-pyrazolinyl, 3-pyrazolinyl, 4-pyrazolinyl, 5-pyrazolinyl, 1-piperidinyl, 2-piperidinyl, 3-piperidinyl, 4-piperidinyl, 2-thiazolidinyl, 3-thiazolidinyl, 4-thiazolidinyl, 1-imidazolidinyl, 2-imidazolidinyl, 4-imidazolidinyl, 5-imidazolidinyl, indolinyl, tetrahydroquinolinyl, tetrahydroisoquinolinyl, benzothiolane, benzodithiane, and 1,3-dihydro-imidazol-2-one.

The term "heteroatom" means one or more of oxygen, sulfur, nitrogen, phosphorus, or silicon (including, any oxidized form of nitrogen, sulfur, phosphorus, or silicon; the quaternized form of any basic nitrogen or; a substitutable nitrogen of a heterocyclic ring, for example N (as in 3,4-dihydro-2H-pyrrolyl), NH (as in pyrrolidinyl) or NR⁺ (as in N-substituted pyrrolidinyl)).

The term "unsaturated", as used herein, means that a moiety has one or more units of unsaturation.

The term "alkoxy", or "thioalkyl", as used herein, refers to an alkyl group, as previously defined, attached to the principal carbon chain through an oxygen ("alkoxy") or sulfur ("thioalkyl") atom.

The terms "haloalkyl", "haloalkenyl" and "haloalkoxy" means alkyl, alkenyl or alkoxy, as the case may be, substituted with one or more halogen atoms. The term "halogen" means F, Cl, Br, or I.

The term "aryl" used alone or as part of a larger moiety as in "aralkyl", "aralkoxy", or "aryloxyalkyl", refers to monocyclic, bicyclic, and tricyclic ring systems having a total of five to fourteen ring members, wherein at least one ring in the system is aromatic and wherein each ring in the system contains 3 to 7 ring members. The term "aryl" may be used interchangeably with the term "aryl ring".

The term "heteroaryl", used alone or as part of a larger moiety as in "heteroaralkyl" or "heteroarylalkoxy", refers to monocyclic, bicyclic, and tricyclic ring systems having a total of five to fourteen ring members, wherein at least one ring in the system is aromatic, at least one ring in the system contains one or more heteroatoms, and wherein each ring in the system contains 3 to 7 ring members. The term "heteroaryl" may be used interchangeably with the term "heteroaryl ring" or the term "heteroaxomatic". Suitable heteroaryl rings include, but are not limited to, 2-furanyl, 3-furanyl, N-imidazolyl, 2-imidazolyl, 4-imidazolyl, 5-imidazolyl, benzimidazolyl, 3-isoxazolyl, 4-isoxazolyl, 5-isoxazolyl, 2-oxazolyl, 4-oxazolyl, 5-oxazolyl, N-pyrrolyl, 2-pyrrolyl, 3-pyrrolyl, 2-pyridyl, 3-pyridyl, 4-pyridyl, 2-pyrimidinyl, 4-pyrimidinyl, 5-pyrimidinyl, pyridazinyl (e.g., 3-pyridazinyl), 2-thiazolyl, 4-thiazolyl, 5-thiazolyl, tetrazolyl (e.g., 5-tetrazolyl), triazolyl (e.g., 2-triazolyl and 5-triazolyl), 2-thienyl, 3-thienyl, benzofuryl, benzothiophenyl, indolyl (e.g., 2-indolyl), pyrazolyl (e.g., 2-pyrazolyl), isothiazolyl, 1,2,3-oxadiazolyl, 1,2,5-oxadiazolyl, 1,2,4-oxadiazolyl, 1,2,3-triazolyl, 1,2,3-thiadiazolyl, 1,3,4-thiadiazolyl, 1,2,5-thiadiazolyl, purinyl, pyrazinyl, 1,3,5-triazinyl, quinolinyl (e.g., 2-quinolinyl, 3-quinolinyl, 4-quinolinyl), and isoquinolinyl (e.g., 1-isoquinolinyl, 3-isoquinolinyl, or 4-isoquinolinyl).

An aryl (including aralkyl, aralkoxy, aryloxyalkyl and the like) or heteroaryl (including heteroaralkyl and heteroarylalkoxy and the like) group may contain one or more substituents and thus may be "optionally substituted". Unless otherwise defined above and herein, suitable substituents on the unsaturated carbon atom of an aryl or heteroaryl group are generally selected from halogen; -R°; -OR°; -SR°; phenyl (Ph) optionally substituted with R°; -O(Ph) optionally substituted with R°; -(CH₂)₁₋₂(Ph), optionally substituted with R°; -CH=CH(Ph), optionally substituted with R°; a 4-6 membered heteroaryl or heterocyclic ring optionally substituted with R°; -NO_{2;} -CN; -N(R°)₂; -NR°C(O)R°; -NR°C(S)R°; -NR°C(O)N(R°)₂; -NR°C(S)N(R°)2; -NR°CO₂R°; -NR°NR°-, -NR°NR°C(O)R°; -NR°NR°C(O)N(R°)₂; -NR°NR°CO₂R°; -C(O)C(O)R°; -C(O)CH₂C(O)R°; -CO₂R°; -C(O)R°; -C(S)R°; -C(O)N(R°)₂; -C(S)N(R°)₂; -OC(O)N(R°)₂; -OC(O)R°; -C(O)N(OR°)R°; -C(NOR°)R°; -S(O)2R°; -S(O)₃R°; -SO₂N(R°)₂; -S(O)R°; -NR°SO₂N(R°)₂; -NR°SO₂R°; -N(OR°)R°; -C(=NH)-N(R°)₂; -C(=NH)-OR°; -P'(O)₂R°; -PO(R°)₂; -OPO(R°)₂; or -(CH₂)₀₋₂NHC(O)R°; wherein each independent occurrence of R° is selected from hydrogen, optionally substituted C₁₋₆ aliphatic, an unsubstituted 4-6 membered heteroaryl or heterocyclic ring, phenyl, -O(Ph), or -CH₂(Ph), or, notwithstanding the definition above, two independent occurrences of R°, on the same substituent or different substituents, taken together with the atom(s) to which each R° group is bound, to form an optionally substituted 3-12 membered saturated, partially unsaturated, or fully unsaturated monocyclic or bicyclic ring having 0-4 heteroatoms independently selected from nitrogen, oxygen, or sulfur.

Optional substituents on the aliphatic group of R° are selected from NH₂, NH(C₁₋₄aliphatic), N(C₁₋₄aliphatic)₂, halogen, C₁₋₄aliphatic, OH, O(C₁₋₄aliphatic), NO₂, CN, CO₂H, CO₂(C₁₋₄aliphatic), O(haloC₁₋₄ aliphatic), or haloC₁₋₄aliphatic, wherein each of the foregoing C₁₋₄aliphatic groups of R° is unsubstituted.

An aliphatic group or a non-aromatic heterocyclic ring may contain one or more substituents and thus may be "optionally substituted". Unless otherwise defined above and herein, suitable substituents on the saturated carbon of an aliphatic or heteroaliphatic group, or of a non-aromatic heterocyclic ring are selected from those listed above for the unsaturated carbon of an aryl or heteroaryl group and additionally include the following: =O, =S, =NNHR*, =NN(R*)₂, =NNHC(O)R*, =NNHCO₂(alkyl), =NNHSO₂(alkyl), =N-OH, =N-(OR*) or =NR*, where each R* is independently selected from hydrogen or an optionally substituted C₁₋₆ aliphatic group.

Unless otherwise defined above and herein, optional substituents on the nitrogen of a non-aromatic heterocyclic ring are generally selected from -R⁺, -N(R⁺)₂, -C(O)R⁺, -CO₂R⁺, -C(O)C(O)R⁺, -C(O)CH₂C(O)R⁺, -SO₂R⁺, -SO₂N(R⁺)₂, -C(=S)N(R⁺¹)₂, -C(=NH)-N(R⁺)2, or -NR⁺SO₂R⁺; wherein R⁺ is hydrogen, an optionally substituted C₁₋₆ aliphatic, optionally substituted phenyl, optionally substituted -O(Ph), optionally substituted -CH₂(Ph), optionally substituted -(CH₂)₁₋₂(Ph); optionally substituted -CH=CH(Ph); or an unsubstituted 4-6 membered heteroaryl or heterocyclic ring having one to four heteroatoms independently selected from oxygen, nitrogen, or sulfur, or, notwithstanding the definition above, two independent occurrences of R⁺, on the same substituent or different substituents, taken together with the atom(s) to which each R⁺ group is bound, form an optionally substituted 3-12 membered saturated, partially unsaturated, or fully unsaturated monocyclic or bicyclic ring having 0-4 heteroatoms independently selected from nitrogen, oxygen, or sulfur.

Optional substituents on the aliphatic group or the phenyl ring of R⁺ are selected from -NH₂, -NH(C₁₋₄ aliphatic), -N(C₁₋₄ aliphatic)₂, halogen, C₁₋₄ aliphatic, -OH, -O(C₁₋₄ aliphatic), -NO₂, -CN, -CO₂H, -CO₂(C₁₋₄ aliphatic), -O(halo C₁₋₄ aliphatic), or halo(C₁₋₄ aliphatic), wherein each of the foregoing C₁₋₄aliphatic groups of R⁺ is unsubstituted.

The term "alkylidene chain" refers to a straight or branched carbon chain that may be fully saturated or have one or more units of unsaturation and has two points of attachment to the rest of the molecule.

The term "protecting group", as used herein, refers to an agent used to temporarily block one or more desired reactive sites in a multifunctional compound. In certain embodiments, a protecting group has one or more, or preferably all, of the following characteristics: a) reacts selectively in good yield to give a protected substrate that is stable to the reactions occurring at one or more of the other reactive sites; and b) is selectively removable in good yield by reagents that do not attack the regenerated functional group. Exemplary protecting groups are detailed in Greene, T.W., Wuts, P. G in "Protective Groups in Organic Synthesis", Third Edition, John Wiley & Sons, New York: 1999, and other editions of this book. The term "nitrogen protecting group", as used herein, refers to an agents used to temporarily block one or more desired nitrogen reactive sites in a multifunctional compound. Preferred nitrogen protecting groups also possess the characteristics exemplified above, and certain exemplary nitrogen protecting groups are also detailed in Chapter 7 in Greene, T.W., Wuts, P. G in "Protective Groups in Organic Synthesis", Third Edition, John Wiley & Sons, New York: 1999, the entire contents of which are hereby incorporated by reference.

In some embodiments, two independent occurrences of a group are taken together with the atom(s) to which they are bound to form a ring. This ring is an optionally substituted 3-12 membered saturated, partially unsaturated, or fully unsaturated monocyclic or bicyclic ring having 0-4 heteroatoms independently selected from nitrogen, oxygen, or sulfur.

Examples of such rings include, but are not limited to the following: piperidin-1-yl, piperazin-1-yl, or morpholin-4-yl group.

Unless otherwise stated, structures depicted herein are also meant to include all enantiomeric, diastereomeric, and geometric (or conformational) forms of the structure; for example, the R and S configurations for each asymmetric center, (Z) and (E) double bond isomers, and (Z) and (E) conformational isomers. Therefore, single stereochemical isomers as well as enantiomeric, diastereomeric, and geometric (or conformational) mixtures of the present compounds are within the scope of the invention.

Unless otherwise stated, all tautomeric forms of the compounds of the invention are within the scope of the invention.

As would be understood by a skilled practitioner, a pyrazole group can be represented in a variety of ways. For example, a structure drawn as 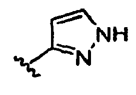 also represents other possible tautomers, such as 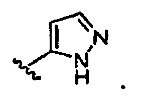 Likewise, a structure drawn as 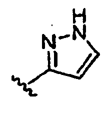 also represents other possible tautomers, such as

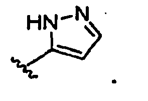

Unless otherwise indicated, a substituent can freely rotate around any rotatable bonds. For example, a substituent drawn as 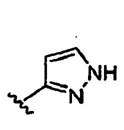 also represents 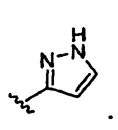 Likewise, a substituent drawn as 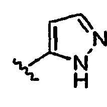 also represents

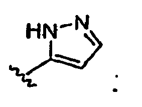

A substituent drawn as 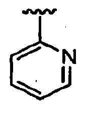 also represents

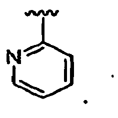

Unless otherwise indicated, a group with two bonds attached at two different ends is attached via the first bond drawn. For example, if J is substituted with -A-X, wherein -A- is -C(=O)O- and X is H, then J is substituted with -C(=O)OH.

Additionally, unless otherwise stated, structures depicted herein are also meant to include compounds that differ only in the presence of one or more isotopically enriched atoms. For example, compounds having the present structures except for the replacement of hydrogen by deuterium or tritium, or the replacement of a carbon by a ¹³C- or ¹⁴C-enriched carbon are within the scope of this invention. Such compounds are useful, for example, as analytical tools or probes in biological assays.

### General Synthetic Methodology

The compounds of this invention may be prepared in light of the specification by methods known to those skilled in the art. Those compounds may be analyzed by known methods, including but not limited to LCMS (liquid chromatography mass spectrometry) and NMR (nuclear magnetic resonance).

It should be understood that the specific conditions shown below are only examples, and are not meant to limit the scope of the conditions that can be used for making compounds of this invention. Instead, this invention also includes conditions that would be apparent to those skilled in that art in light of this specification for making the compounds of this invention. Starting materials shown are either commercially available or can be readily accessible from methods known to one skilled in the art. Unless otherwise indicated, all variables in the following schemes are as defined herein.

The following abbreviations are used:
DIPEA is diisopropylethylamine
DMF is dimethylformamide
i-PrOH is isopropyl alcohol
n-BuOH is n-butanol
t-BuOH is tert-butanol
EtOH is ethanol
MeOH is methanol
EtOAc is ethyl acetate
TFA is trifluoroacetic acid
DMSO is dimethyl sulfoxide
Rt is retention time
DCM is dichloromethane
MeCN is acetonitrile
THF is tetrahydrofuran
TBTU is 2-(1H-Benzotriazole-1-yl)-1,1,3,3-tetramethyluronium tetrafluoroborate
HPLC is high performance liquid chromatography LCMS liquid chromatography mass spectrometry ¹H NMR is nuclear magnetic resonance

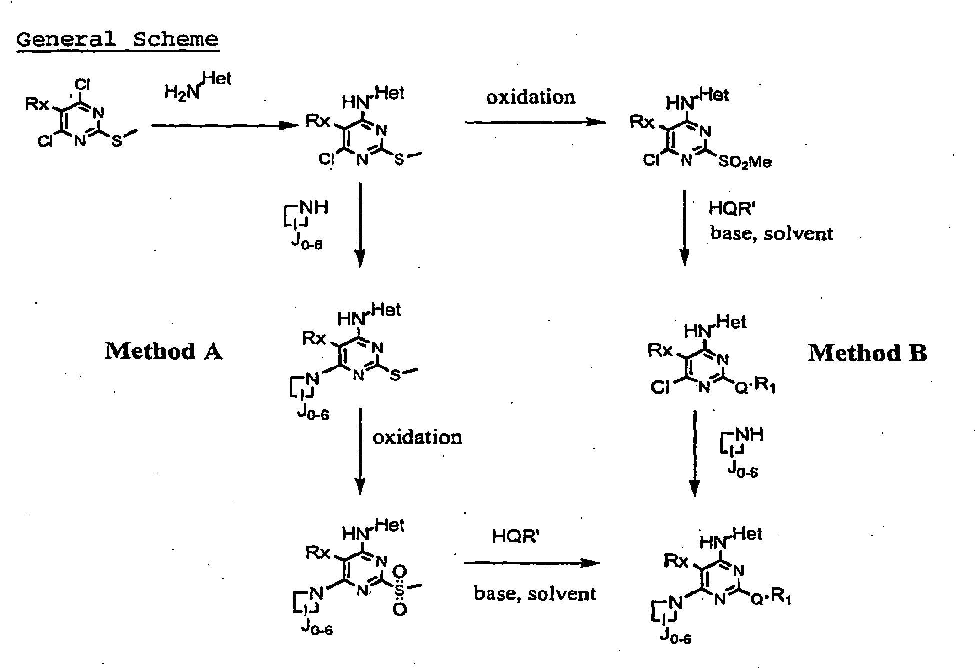

The general scheme above shows some methods of making compounds of this invention wherein the azetidine is directly attached via a nitrogen atom.

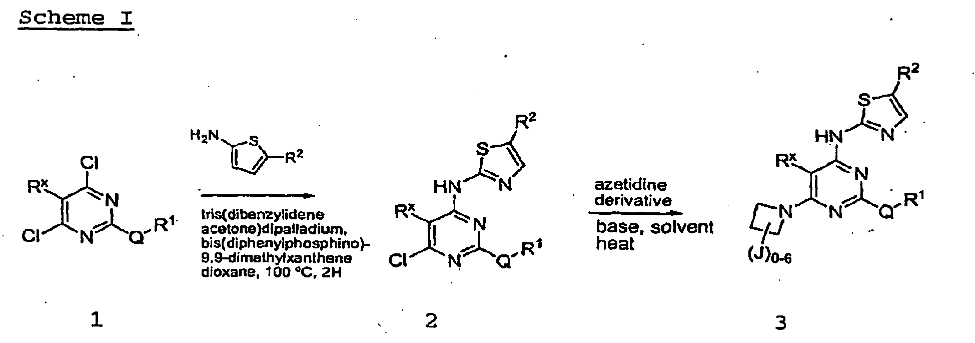

Scheme I above shows a general route for the preparation of compounds of formula 3, wherein R^{x}, R¹, R², and J are as defined herein and Q is -O-, -NR'-, or -S-. As shown above, the dichlorinated pyrimidine of formula 1 is heated in the presence of a suitable palladium catalyst and a suitable solvent (e.g. dioxane) with an optionally substituted aminothiazole to form a compound of formula 2. Alternatively, the dichlorinated pyrimidine of formula 1 can be heated in the presence of a suitable base (e.g. NaI/DIPEA) and a suitable solvent (e.g. DMF) with an optionally substituted aminothiazole to form a compound of formula 2. The compound of formula 2 is then heated in the presence of a suitable base (e.g. DIPEA/NaI) and a suitable solvent, (e.g. n-BuOH), with an azetidine derivative to form the compound of formula 3.

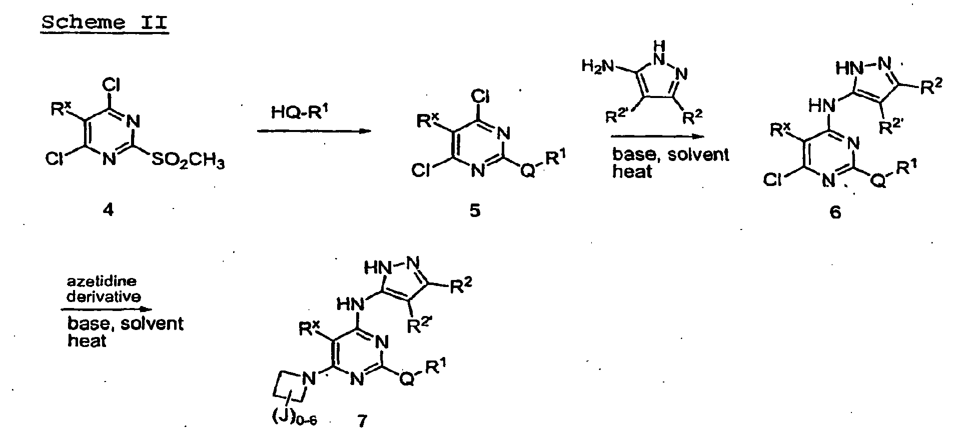

Scheme II above shows a general route for the preparation of compounds of formula 7, wherein R^{x}, R¹, R², R^{2'}, and J are as defined herein and Q is -O-, -NR'-, or -S-. The dichlorinated pyrimidine of formula 4 is combined with HQ-R¹ to form a compound of formula 5. In some embodiments, the two compounds are heated in the presence of a suitable solvent (e.g. t-BuOH) for 16 hours. In other embodiments, the two compounds are mixed at 0 ºC in the presence of acetonitrile and triethylamine for 1 hour. The compound of formula 5 is then heated in the presence of a suitable solvent (e.g. DMF) and a suitable base (e.g. DIPEA/NaI) with an optionally substituted aminopyrazole to form the compound of formula 6, which is heated in the presence of an azetidine derivative in the presence of a suitable solvent (e.g. n-BuOH) to form the compound of formula 7.

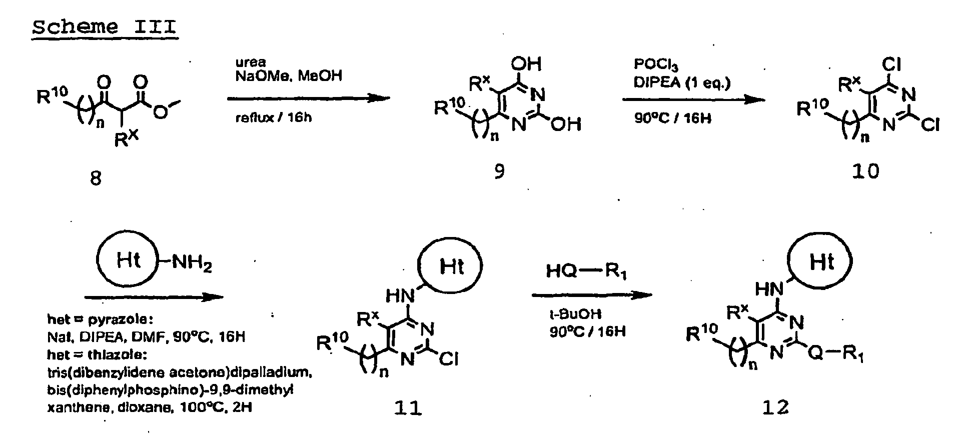

Scheme III above shows a general route for the preparation of compounds of formula 12, wherein R^{x}, R¹, R¹⁰, and Ht are as defined herein and Q is -O-, -NR'-, or -S-. The ketoester of formula 8 is cyclized in the presence of urea, a suitable solvent (e.g. MeOH, EtOH), and a suitable base (e.g. NaOMe) to form the dihydroxy pyrimidine of formula 9. The compound of formula 9 is then chlorinated under suitable chlorination conditions, such as heating in the presence of POCl₃ and DIPEA, to form the dichloropyrimidine of formula 10. The dichloropyrimidine is then heated in the presence of an appropriate amino-heteroaryl under suitable conditions known to those skilled in the art (see scheme for examples) to form a compound of formula 11, which is subsequently heated with HQ-R¹ in the presence of a suitable solvent (e.g. t-BuOH) to form the compound of formula 12.

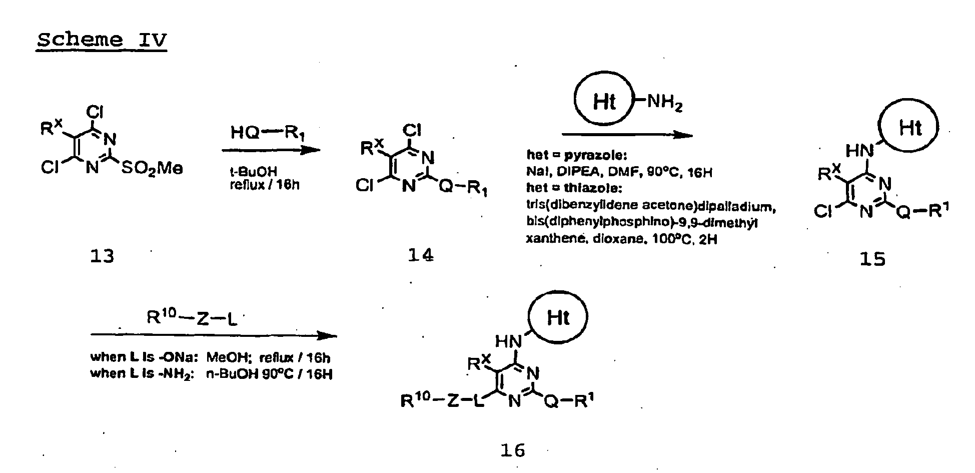

Scheme IV above shows a general route for the preparation of compounds of formula 16, wherein L is an appropriate nucleophile (such as N, O, or S), Q is -O-, -NR'-, or -S-, and Z, R¹, R¹⁰, and Ht are as defined herein. The dichloropyrimidine of formula 13 is heated in the presence of HQ-R¹ to form the substituted dichloropyrimidine of formula 14. The compound of formula 14 is then heated in the presence of an appropriate amino-heteroaryl under suitable conditions known to those skilled in the art (see scheme for examples) to form a compound of formula 15, which is heated with R¹⁰-Z-L, wherein L is an appropriate nucleophile (such as N, O, or S), and Z and R¹⁰ are as defined herein, to form a compound of formula 16.

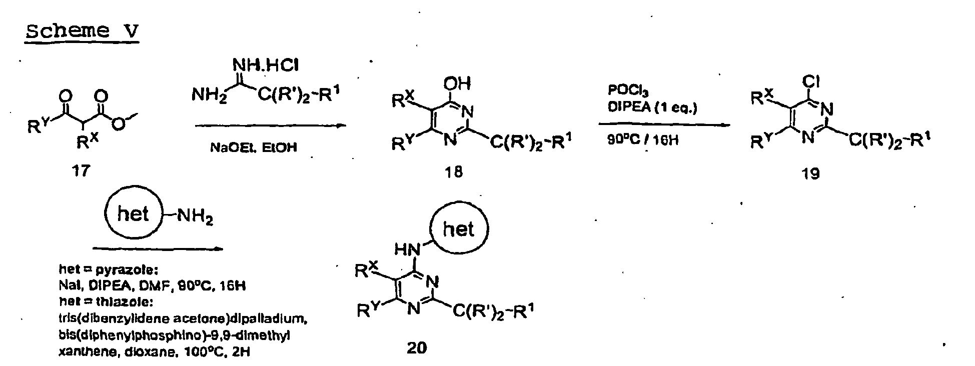

Scheme V above shows a general route for the preparation of compounds of formula 20, wherein R¹, R^{X}, R^{Y}, R^{'} and Ht are as defined herein. The ketoester of formula 17, together with a substituted amidine, cyclizes to form the hydroxypyrimidine of formula 18. The compound of formula 18 is then chlorinated under suitable chlorination conditions known to those skilled in the art (e.g.POCl₃/DIPEA), to form the chloropyrimidine of formula 19. The chloropyrimidine is then heated in the presence of an appropriate amino-heteroaryl under suitable conditions known to those skilled in the art (see scheme for examples) to form a compound of formula 20.

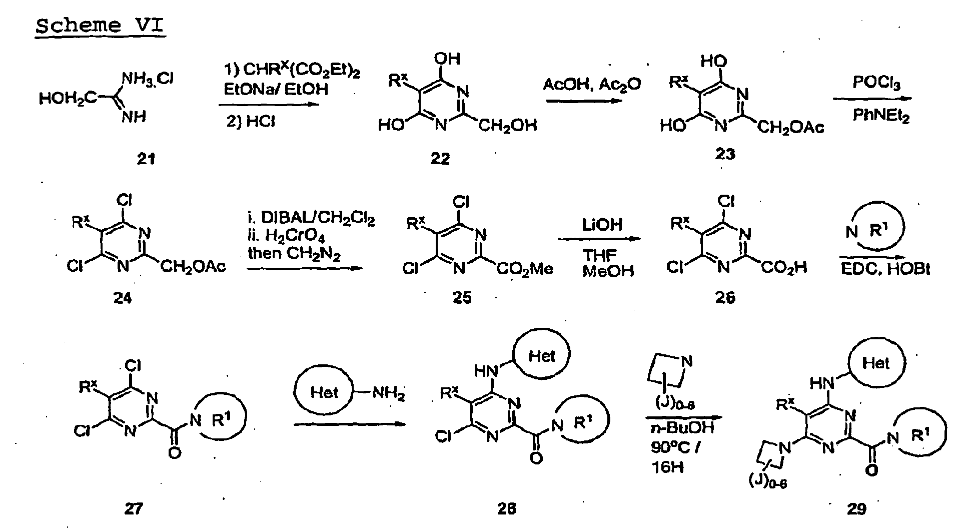

Scheme VI above shows a general route for the preparation of compounds of formula 29, wherein R^{X}, Het, and J are as defined herein, Q is -(C=O)-, and R¹ is an optionally substituted heterocyclyl. The amidine of formula 21 is cyclized with a malonic ester in the presence of a base to form the desired dihydroxypyrimidine of formula 22. The compound of formula 22 is then acetylated under suitable conditions (such as AcOH, AC₂O) to form a compound of formula 23. The compound of formula 23 is then chlorinated according to methods known to one skilled in the art (such as POCl₃), to form the dichloropyrimidine of formula 24. The compound of formula 24 is then reduced and then oxidized in the presence of suitable reducing and oxidizing agents (see scheme for examples) to form the resultant carboxylic ester (formula 25), which is then hydrolyzed to the carboxylic acid (formula 26) under suitable hydrolyzing conditions (e.g. LiOH, THF, MeOH). The acid of formula 26 is then coupled with an appropriate amine under suitable peptide coupling conditions (e.g. EDC, HOBt) to form the amide of formula 27. The amide of formula 27 is then combined with the desired heteroaryl-amine to form the compound of formula 28. In some embodiments, when the heteroaryl is a pyrazole, the conditions are NaI, DIPEA, DMF, 90° C for 16H. In other embodiments, when the heteroaryl is thiazole, the conditions are tris(dibenzylideneacetone) dipalladium, bis(diphenylphosphino)-9,9-dimethylxanthene, dioxane, 100°C, for 2H. The compound of formula 28 is then heated with an optionally substituted azetidine to form a compound of formula 29.

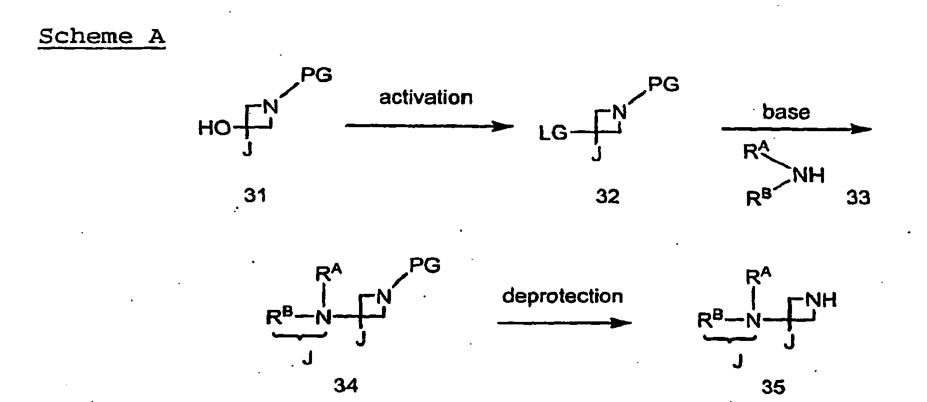

Scheme A above shows a general route for the preparation of N-substituted azetidines wherein at least one J group is bonded to the azetidine via a nitrogen atom. Protected azetidine 31 is activated with a suitable leaving group under suitable conditions to form azetidine 32, which, upon treatment with NHR^{A}R^{B} under basic conditions, forms the amine-substituted azetidine 34. Azetidine 34 is then deprotected under suitable nitrogen deprotection conditions to form compound 35.

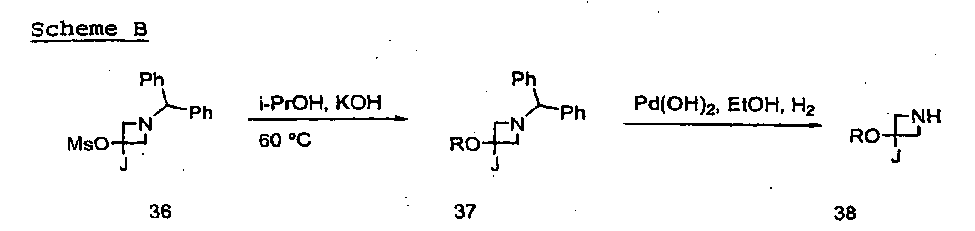

Scheme B above shows a general route for the preparation of O-substituted azetidines wherein at least one J group is OR wherein R is H or C₁₋₆alkyl.

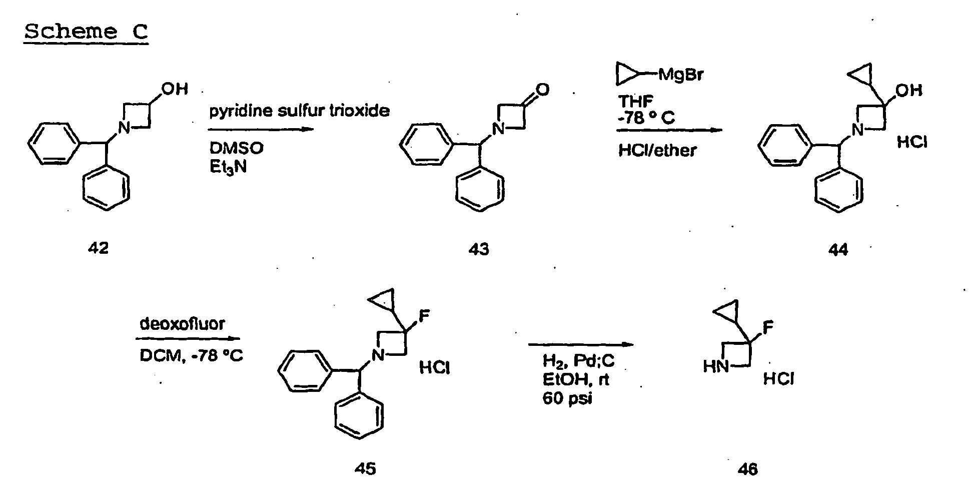

Scheme C depicts a general route for the preparation of cyclopropyl-fluoro-substituted azetidines. Compound 42 is oxidized under suitable conditions to form compound 43, which, under suitable Grignard conditions, is combined with cyclopropyl-MgBr to form the cyclopropyl-substituted azetidine 44. Compound 44 is then fluorinated under suitable fluorination conditions to form 45, which is hydrogenated under Pd/C conditions to form the deprotected free azetidine 46.

Accordingly, this invention relates to processes for making the compounds of this invention.

One aspect of this invention relates to a method for treating a disease state in patients that is alleviated by treatment with a protein kinase inhibitor, which method comprises administering to a patient in need of such a treatment a therapeutically effective amount of a compound of formula I (herein including Ia and Ib). The method is particularly useful for treating a disease state that is alleviated by the use of an inhibitor of a kinase such as the Aurora kinases (Aurora A, Aurora B, Aurora C), FLT-3, JAK-2, JAK-3, ITK, Src, Abl, Abl(T315I), Arg, FGFR1, MELK, MLK1, MuSK, Ret, TrkA, PLK4, Tie-2, and TrkA.

The activity of the compounds as protein kinase inhibitors may be assayed *in vitro, in vivo* or in a cell line. *In vitro* assays include assays that determine inhibition of either the kinase activity or ATPase activity of the activated kinase. Alternate *in vitro* assays quantitate the ability of the inhibitor to bind to the protein kinase and may be measured either by radiolabelling the inhibitor prior to binding, isolating the inhibitor/kinase complex and determining the amount of radiolabel bound, or by running a competition experiment where new inhibitors are incubated with the kinase bound to known radioligands.

Another aspect of this invention is directed towards a method of treating cancer in a subject in need thereof, comprising the sequential or co-administration of a compound of this invention or a pharmaceutically acceptable salt thereof, and another therapeutic agent. In some embodiments, said additional therapeutic agent is selected from an anticancer agent, an anti-proliferative agent, or a chemotherapeutic agent.

In some embodiments, said additional therapeutic agent is selected from camptothecin, the MEK inhibitor: U0126, a KSP (kinesin spindle protein) inhibitor, adriamycin, interferons, and platinum derivatives, such as Cisplatin.

In other embodiments, said additional therapeutic agent is selected from taxanes; inhibitors of bcr-abl (such as Gleevec, dasatinib, and nilotinib); inhibitors of EGFR (such as Tarceva and Iressa); DNA damaging agents (such as cisplatin, oxaliplatin, carboplatin, topoisomerase inhibitors, and anthracyclines); and antimetabolites (such as AraC and 5-FU).

In yet other embodiments, said additional therapeutic agent is selected from camptothecin, doxorubicin, idarubicin, Cisplatin, taxol, taxotere, vincristine, tarceva, the MEK inhibitor, U0126, a KSP inhibitor, vorinostat, Gleevec, dasatinib, and nilotinib.

In another embodiment, said additional therapeutic agent is selected from Her-2 inhibitors (such as Herceptin); HDAC inhibitors (such as vorinostat), VEGFR inhibitors (such as Avastin), c-KIT and FLT-3 inhibitors (such as sunitinib), BRAF inhibitors (such as Bayer's BAY 43-9006) MEK inhibitors (such as Pfizer's PD0325901); and spindle poisons (such as Epothilones and paclitaxel protein-bound particles (such as Abraxane^{®}). In another embodiment said additional therapeutic agent is selected from taxanes, inhibitors of bcr-abl, inhibitors of EGFR, DNA damaging agents, and antimetabolites. In another embodiment said additional therapeutic agent is selected from Paclitaxel, Gleevec, dasatinib, nilotinib, Tarceva, Iressa, cisplatin, oxaliplatin, carboplatin, anthracyclines, AraC and 5-FU. In another embodiment said additional therapeutic agent is selected from camptothecin, doxorubicin, idarubicin, Cisplatin, taxol, taxotere, vincristine, tarceva, the MEK inhibitor, U0126, a KSP inhibitor, vorinostat, Gleevec, dasatinib, and nilotinib.

Other therapies or anticancer agents that may be used in combination with the inventive agents of the present invention include surgery, radiotherapy (in but a few examples, gamma-radiation, neutron beam radiotherapy,

electron beam radiotherapy, proton therapy, brachytherapy, and systemic radioactive isotopes, to name a few), endocrine therapy, biologic response modifiers (interferons, interleukins, and tumor necrosis factor (TNF) to name a few), hyperthermia and cryotherapy, agents to attenuate any adverse effects (e.g., antiemetics), and other approved chemotherapeutic drugs, including, but not limited to, alkylating drugs (mechlorethamine, chlorambucil, Cyclophosphamide, Melphalan, Ifosfamide), antimetabolites (Methotrexate), purine antagonists and pyrimidine antagonists (6-Mercaptopurine, 5-Fluorouracil, Cytarabile, Gemcitabine), spindle poisons (Viriblastine, Vincristine, Vinorelbine, Paclitaxel), podophyllotoxins (Etoposide, Irinotecan, Topotecan), antibiotics (Doxorubicin, Bleomycin, Mitomycin), nitrosoureas (Carmustine, Lomustine), inorganic ions (Cisplatin, Carboplatin), enzymes (Asparaginase), and hormones (Tamoxifen, Leuprolide, Flutamide, and Megestrol), Gleevec™, adriamycin, dexamethasone, and cyclophosphamide.

A compound of the instant invention may also be useful for treating cancer in combination with the following therapeutic agents: abarelix (Plenaxis depot^{®}); aldesleukin (Prokine^{®}); Aldesleukin (Proleukin^{®}); Alemtuzumabb (Campath^{®}); alitretinoin (Panretin^{®}); allopurinol (zyloprim^{®}); altretamine (Hexalen^{®}); amifostine (Ethyol^{®}); anastrozole (Arimidex^{®}); arsenic trioxide (Trisenox^{®}); asparaginase (Elspar^{®}); azacitidine (vidaza^{®}); bevacuzimab (Avastin^{®}); bexarotene capsules (Targretin^{®}); bexarotene gel (Targretin^{®}); bleomycin (Blenoxane^{®}); bortezomib (Velcade^{®}); busulfan intravenous (Busulfex^{®}); busulfan oral (Myleran^{®}); calusterone (Methosarb^{®}); capecitabine (Xeloda^{®}); carboplatin (Paraplatin^{®}); carmustine (BCNU^{®}, BiCNU^{®}); carmustine (Gliadel^{®}); carmustine with Polifeprosan 20 Implant (Gliadel Wafer^{®}); celecoxib (Celebrex^{®}); cetuximab (Erbitux^{®}); chlorambucil (Leukeran^{®}); cisplatin (Platinol^{®}); cladribine (Leustatin^{®}, 2-CdA^{®}); clofarabine (Clolar^{®}); cyclophosphamide (Cytoxan^{®}, Neosar^{®}); cyclophosphamide (Cytoxan Injection^{®}); cyclophosphamide (Cytoxan Tablet^{®}); cytarabine (Cytosar-U^{®}); cytarabine liposomal (DepoCyt^{®}); dacarbazine (DTIC-Dome^{®}); dactinomycin, actinomycin D (Cosmegen^{®}); Darbepoetin alfa (Aranesp^{®}); daunorubicin liposomal (DanuoXome^{®}); daunorubicin, daunomycin (Daunorubicin^{®}); daunorubicin, daunomycin (Cerubidine^{®}); Denileukin diftitox (Ontak^{®}); dexrazoxane (Zinecard^{®}); docetaxel (Taxotere^{®}); doxorubicin (Adriamycin PFS^{®}); doxorubicin (Adriamycin^{®}, Rubex^{®}); doxorubicin (Adriamycin PFS Injection^{®}); doxorubicin liposomal (Doxil^{®}); dromostanolone propionate (dromostanolone^{®}); dromostanolone propionate (masterone injection^{®}); Elliott's B Solution (Elliott's B Solution^{®}); epirubicin (Ellence^{®}); Epoetin alfa (epogen^{®}); erlotinib (Tarceva^{®}); estramustine (Emcyt^{®}); etoposide phosphate (Etopophos^{®}); etoposide, VP-16 (Vepesid^{®}); exemestane (Aromasin^{®}); Filgrastim (Neupogen^{®}); floxuridine (intraarterial) (FUDR^{®}); fludarabine (Fludara^{®}); fluorouracil, 5-FU (Adrucil^{®}); fulvestrant (Faslodex^{®}); gefitinib (Iressa^{®}); gemcitabine (Gemzar^{®}); gemtuzumab ozogamicin (mylotarg^{®}); goserelin acetate (Zoladex Implant^{®}); goserelin acetate (Zoladex^{®}); histrelin acetate (Histrelin implant^{®}); hydroxyurea (Hydrea^{®}); Ibritumomab Tiuxetan (Zevalin^{®}); idarubicin (Idamycin^{®}); ifosfamide (IFEX^{®}); imatinib mesylate (Gleevec^{®}); interferon alfa 2a (Roferon A^{®}); Interferon alfa-2b (Intron A^{®}); irinotecan (Camptosar^{®}); lenalidomide (Revlimid^{®}); letrozole (Femara^{®}); leucovorin (Wellcovorin^{®}, Leucovorin^{®}); Leuprolide Acetate (Eligard^{®}); levamisole (Ergamisol^{®}); lomustine, CCNU (CeeBU^{®}); meclorethamine, nitrogen mustard (Mustargen^{®}); megestrol acetate (Megace^{®}); melphalan, L-PAM (Alkeran^{®}); mercaptopurine, 6-MP (Purinethol^{®}); mesna (Mesnex^{®}); mesna (Mesnex tabs^{®}); methotrexate (Methotrexate^{®}); methoxsalen (Uvadex^{®}); mitomycin C (Mutamycin^{®}); mitotane (Lysodren^{®}); mitoxantrone (Novantrone^{®}); nandrolone phenpropionate (Durabolin-50^{®}); nelarabine (Arranon^{®}); Nofetumomab (Verluma^{®}); Oprelvekin (Neumega^{®}): oxaliplatin (Eloxatin^{®}); paclitaxel (Paxene^{®}); paclitaxel (Taxol^{®}); paclitaxel protein-bound particles (Abraxane^{®}); palifermin, (Kepivance^{®}); pamidronate (Aredia^{®}); pegademase (Adagen (Pegademase Bovine)^{®}); pegaspargase (Oncaspar^{®}); Pegfilgrastim (Neulasta^{®}); pemetrexed disodium (Alimta^{®}); pentostatin (Nipent^{®}); pipobroman (Vercyte^{®}); plicamycin, mithramycin (Mithracin®); porfimer sodium (Photofrin^{®}); procarbazine (Matulane^{®}); quinacrine (Atabrine^{®}); Rasburicase (Elitek^{®}); Rituximab (Rituxan^{®}); sargramostim (Leukine^{®}); Sargramostim (Prokine^{®}); sorafenib (Nexavar^{®}); streptozocin (zanosar^{®}); sunitinib maleate (Sutent^{®}); talc (Sclerosol^{®}); tamoxifen (Nolvadex^{®}); temozolomide (Ternodar^{®}); teniposide, VM-26 (Vumon^{®}); testolactone (Teslac^{®}); thioguanine, 6-TG (Thioguanine^{®}); thiotepa (Thioplex^{®}); topotecan (Hycamtin^{®}); toremifene (Fareston^{®}); Tositumomab (Bexxar^{®}); Tositumomab/I-131 tositumomab (Bexxar^{®}); Trastuzumab (Herceptin^{®}); tretinoin, ATRA (Vesanoid^{®}); Uracil Mustard (Uracil Mustard Capsules^{®}); valrubicin (Valstar^{®}); vinblastine (Velban^{®}); vincristine (Oncovin^{®}); vinorelbine (Navelbine^{®}); zoledronate (Zometa^{®}) and vorinostat (Zolinza^{®}).

For a comprehensive discussion of updated cancer therapies see, http://www.nci.nih.gov/, a list of the FDA approved oncology drugs at http://www.fda.gov/cder/cancer/druglistframe.htm, and The Merck Manual, Seventeenth Ed. 1999.

The protein kinase inhibitors or pharmaceutical salts thereof may be formulated into pharmaceutical compositions for administration to animals or humans. These pharmaceutical compositions, which comprise an amount of the protein inhibitor effective to treat or prevent kinase mediated condition and a pharmaceutically acceptable carrier, are another embodiment of the present invention.

The term "protein kinase-mediated condition", as used herein, diseases or other deleterious conditions in which a protein kinase is known to play a role. Such conditions include, without limitation, autoimmune diseases, inflammatory diseases, neurological and neurodegenerative diseases, cancer, cardiovascular diseases, allergy and asthma. The term "cancer" includes, but is not limited to the following cancers: breast; ovary; cervix; prostate; testis, genitourinary tract; esophagus; larynx, glioblastoma; neuroblastoma; stomach; skin, keratoacanthoma; lung, epidermoid carcinoma, large cell carcinoma, small cell carcinoma, lung adenocarcinoma; bone; colon, adenoma; pancreas, adenocarcinoma; thyroid, follicular carcinoma, undifferentiated carcinoma, papillary carcinoma; seminoma; melanoma; sarcoma; bladder carcinoma; liver carcinoma and biliary passages; kidney carcinoma; myeloid disorders; lymphoid disorders, Hodgkin's, hairy cells; buccal cavity and pharynx (oral), lip, tongue, mouth, pharynx; small intestine; colon-rectum, large intestine, rectum; brain and central nervous system; and leukemia.

The term "cancer" also includes, but is not limited to, the following cancers: epidermoid Oral: buccal cavity, lip, tongue, mouth, pharynx; Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell or epidermoid, undifferentiated small cell, undifferentiated large cell, non-small cell, adenocarcinoma, alveolar (bronchiolar) carcinoma, bronchial adenoma, sarcoma, lymphoma, chondromatous hamartoma, mesothelioma; Gastrointestinal: esophagus (squamous cell carcinoma, larynx, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel or small intestines (adenocarcinoma, lymphoma, carcinoid tumors, Karposi's sarcoma, leiomyoma, hemangioma, lipoma, neurofibroma, fibroma), large bowel or large intestines (adenocarcinoma, tubular adenoma, villous adenoma, hamartoma, leiomyoma), colon, colon-rectum, colorectal; rectum, Genitourinary tract: kidney (adenocarcinoma, Wilm's tumor [nephroblastoma], lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma, biliary passages; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma); Gynecological: uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma), breast; Hematologic: blood (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma] hairy cell; lymphoid disorders; Skin: malignant melanoma, basal cell carcinoma, squamous cell carcinoma, Karposi's sarcoma, keratoacanthoma, moles dysplastic nevi, lipoma, angioma, dermatofibroma, keloids, psoriasis, Thyroid gland: papillary thyroid carcinoma, follicular thyroid carcinoma; medullary thyroid carcinoma, undifferentiated thyroid cancer, multiple endocrine neoplasia type 2A, multiple endocrine neoplasia type 2B, familial medullary thyroid cancer, pheochromocytoma, paraganglioma; and Adrenal glands: neuroblastoma. Thus, the term "cancerous cell" as provided herein, includes a cell afflicted by any one of the above-identified conditions. In some embodiments, the cancer is selected from colorectal, thyroid, or breast cancer.

The term "Aurora-mediated condition" or "Aurora-mediated disease" as used herein means any disease or other deleterious condition in which Aurora (Aurora A, Aurora B, and Aurora C) is known to play a role. Such conditions include, without limitation, cancer such as colorectal, thyroid, and breast cancer; and myeloproliferative disorders, such as polycythemia vera, thrombocythemia, myeloid metaplasia with myelofibrosis, chronic myelogenous leukaemia (CML), chronic myelomonocytic leukemia, hypereosinophilic syndrome, juvenile myelomonocytic leukemia, and systemic mast cell disease.

The compounds of this invention are useful for treating cancer, such as colorectal, thyroid, breast, and non-small cell lung cancer; and myeloproliferative disorders, such as polycythemia vera, thrombocythemia, myeloid metaplasia with myelofibrosis, chronic myelogenous leukemia, chronic myelomonocytic leukemia, hypereosinophilic syndrome, juvenile myelomonocytic leukemia, and systemic mast cell disease.

The compounds of this invention are useful for treating hematopoietic disorders, in particular, acute-myelogenous leukemia (AML), chronic-myelogenous leukemia (CML), acute-promyelocytic leukemia (APL), and acute lymphocytic leukemia (ALL).

In addition to the compounds of this invention, pharmaceutically acceptable derivatives or prodrugs of the compounds of this invention may also be employed in compositions to treat or prevent the above-identified disorders.

A "pharmaceutically acceptable derivative or prodrug" means any pharmaceutically acceptable salt, ester, salt of an ester or other derivative of a compound of this invention which, upon administration to a recipient, is capable of providing, either directly or indirectly, a compound of this invention or an inhibitorily active metabolite or residue thereof. Particularly favored derivatives or prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

Pharmaceutically acceptable prodrugs of the compounds of this invention include, without limitation, esters, amino acid esters, phosphate esters, metal salts and sulfonate esters.

The compounds of this invention can exist in free form for treatment, or where appropriate, as a pharmaceutically acceptable salt.

As used herein, the term "pharmaceutically acceptable salt" refers to salts of a compound which are, within the scope of sound medical judgment, suitable for use in contact with the tissues of humans and lower animals without undue toxicity, irritation, allergic response and the like, and are commensurate with a reasonable benefit/risk ratio.

Pharmaceutically acceptable salts of the compounds of this invention include those derived from pharmaceutically acceptable inorganic and organic acids and bases. These salts can be prepared *in situ* during the final isolation and purification of the compounds. Acid addition salts can be prepared by 1) reacting the purified compound in its free-based form with a suitable organic or inorganic acid and 2) isolating the salt thus formed.

Examples of suitable acid salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, formate, fumarate, glucoheptanoate, glycerophosphate, glycolate, hemisulfate, heptanoate, hexanoate, hydrochloride, hydrobromide, hydroiodide, 2-hydroxyethanesulfonate, lactate, maleate, malonate, methanesulfonate, 2-naphthalenesulfonate, nicotinate, nitrate, oxalate, palmoate, pectinate, persulfate, 3-phenylpropionate, phosphate, picrate, pivalate, propionate, salicylate, succinate, sulfate, tartrate, thiocyanate, tosylate and undecanoate. Other acids, such as oxalic, while not in themselves pharmaceutically acceptable, may be employed in the preparation of salts useful as intermediates in obtaining the compounds of the invention and their pharmaceutically acceptable acid addition salts.

Base addition salts can be prepared by 1) reacting the purified compound in its acid form with a suitable organic or inorganic base and 2) isolating the salt thus formed.

Salts derived from appropriate bases include alkali metal (e.g., sodium and potassium), alkaline earth metal (e.g., magnesium), ammonium and N⁺(C₁₋₄ alkyl)₄ salts. This invention also envisions the quaternization of any basic nitrogen-containing groups of the compounds disclosed herein. Water or oil-soluble or dispersible products may be obtained by such quaternization.

Base addition salts also include alkali or alkaline earth metal salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate. Other acids and bases, while not in themselves pharmaceutically acceptable, may be employed in the preparation of salts useful as intermediates in obtaining the compounds of the invention and their pharmaceutically acceptable acid or base addition salts.

Pharmaceutically acceptable carriers that may be used in these pharmaceutical compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intraperitoneal, intrahepatic, intralesional and intracranial injection or infusion techniques.

Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a nontoxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

The pharmaceutical compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, the pharmaceutical compositions of this invention may be administered in the form of suppositories for rectal administration. These can be prepared by mixing the agent with a suitable non-irritating excipient which is solid at room temperature but liquid at rectal temperature and therefore will melt in the rectum to release the drug. Such materials include cocoa butter, beeswax and polyethylene glycols.

The pharmaceutical compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutical compositions may be formulated in an ointment such as petrolatum.

The pharmaceutical compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

The amount of kinase inhibitor that may be combined with the carrier materials to produce a single dosage form will vary depending upon the host treated, the particular mode of administration. Preferably, the compositions should be formulated so that a dosage of between 0.01 - 100 mg/kg body weight/day of the inhibitor can be administered to a patient receiving these compositions.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of inhibitor will also depend upon the particular compound in the composition.

The compounds of the invention may be used for treating or preventing a kinase-mediated condition comprising the step of administering to a patient one of the above-described pharmaceutical compositions. The term "patient", as used herein, means an animal, preferably a human.

Said kinase-mediated condition may be a proliferative disorder or cancer. Said kinase-mediated condition may be selected from a hematopoietic disorder, in particular, acute-myelogenous leukemia (AML), acute-promyelocytic leukemia (APL), chronic-myelogenous leukemia (CML), and acute lymphocytic leukemia (ALL).

Preferably the compounds of the invention may be used to treat or prevent a condition selected from cancers such as cancers of the breast, colon, prostate, skin, pancreas, brain, genitourinary tract, lymphatic system, stomach, larynx and lung, including lung adenocarcinoma and small cell lung cancer; stroke, diabetes, myeloma, hepatomegaly, cardiomegaly, Alzheimer's disease, cystic fibrosis, and viral disease, or any specific disease or disorder described above.

The compounds of the invention may be used for treating or preventing cancer, a proliferative disorder, or a myeloproliferative disorder comprising the step of administering to a patient one of the herein-described compounds or pharmaceutical compositions.

The compounds of the invention may be used to treat or prevent a hematopoietic disorder, such as acute-myelogenous leukemia (AML), acute-promyelocytic leukemia (APL), chronic-myelogenous leukemia (CML), or acute lymphocytic leukemia (ALL).

The compounds of the invention may be used to treat or prevent myeloproliferative disorders, such as polycythemia vera, thrombocythemia, myeloid metaplasia with myelofibrosis, chronic myelogenous leukaemia (CML), chronic myelomonocytic leukemia, hypereosinophilic syndrome, juvenile myelomonocytic leukemia, and systemic mast cell disease.

The compounds of the invention may be used to treat or prevent cancer, such as cancers of the breast, colon, prostate, skin, pancreas, brain, genitourinary tract, lymphatic system, stomach, larynx and lung, including lung adenocarcinoma, small cell lung cancer, and non-small cell lung cancer.

The compounds of the invention may be used to inhibit kinase activity in a patient. Said kinase may be an Aurora kinase (Aurora A, Aurora B, Aurora C), FLT-3, JAK-2, JAK-3, ITK, Src, Abl, Abl(T315I), Arg, FGFR1, MELK, MLK1, MuSK, Ret, TrkA, PLK4, Tie-2, and TrkA.

The compounds of the invention may be used in a method for inhibiting kinase activity in a biological sample, which method comprises contacting said biological sample with a compound of formula **I** or a composition comprising said compound. The term "biological sample", as used herein, means an in vitro or an ex vivo sample, including, without limitation, cell cultures or extracts thereof; biopsied material obtained from a mammal or extracts thereof; and blood, saliva, urine, feces, semen, tears, or other body fluids or extracts thereof.

Inhibition of kinase activity in a biological sample is useful for a variety of purposes that are known to one of skill in the art. Examples of such purposes include, but are not limited to, blood transfusion, organ-transplantation, biological specimen storage, and biological assays.

Another aspect of this invention relates to the study of kinases in biological and pathological phenomena; the study of intracellular signal transduction pathways mediated by such kinases; and the comparative evaluation of new kinase inhibitors. Examples of such uses include, but are not limited to, biological assays such as enzyme assays and cell-based assays.

Depending upon the particular conditions to be treated or prevented, additional drugs, which are normally administered to treat or prevent that condition, may be administered together with the inhibitors of this invention. For example, chemotherapeutic agents or other anti-proliferative agents may be combined with the inhibitors of this invention to treat proliferative diseases.

Examples of known chemotherapeutic agents include, but are not limited to, Gleevec®, adriamycin, dexamethasone, vincristine, cyclophosphamide, fluorouracil, topotecan, taxol, interferons, and platinum derivatives.

Other examples of agents the compounds of this invention may also be combined with include, without limitation: treatments for Alzheimer's Disease such as Aricept^{®} and Excelon^{®}; treatments for Parkinson's Disease such as L-DOPA/carbidopa, entacapone, ropinrole, pramipexole, bromocriptine, pergolide, trihexephendyl, and amantadine; agents for treating Multiple Sclerosis (MS) such as beta interferon (e.g., Avonex^{®} and Rebif^{®}), Copaxone^{®}, and mitoxantrone; treatments for asthma such as albuterol and Singulair^{®}; agents for treating schizophrenia such as zyprexa, risperdal, seroquel, and haloperidol; anti-inflammatory agents such as corticosteroids, TNF-blockers, IL-1 RA, azathioprine, cyclophosphamide, and sulfasalazine; immunomodulatory and immunosuppressive agents such as cyclosporin, tacrolimus, rapamycin, mycophenolate mofetil, interferons, corticosteroids, cyclophophamide, azathioprine, and sulfasalazine; neurotrophic factors such as acetylcholinesterase inhibitors, MAO inhibitors, interferons, anti-convulsants, ion channel blockers, riluzole, and anti-Parkinsonian agents; agents for treating cardiovascular disease such as beta-blockers, ACE inhibitors, diuretics, nitrates, calcium channel blockers, and statins; agents for treating liver disease such as corticosteroids, cholestyramine, interferons, and anti-viral agents; agents for treating blood disorders such as corticosteroids, anti-leukemic agents, and growth factors; and agents for treating immunodeficiency disorders such as gamma globulin. Another embodiment provides a simultaneous, separate or sequential use of a combined preparation.

Those additional agents may be administered separately, as part of a multiple dosage regimen, from the kinase inhibitor-containing compound or composition. Alternatively, those agents may be part of a single dosage form, mixed together with the kinase inhibitor in a single composition.

Methods for evaluating the activity of the compounds of this invention (e.g., kinase assays) are known in the art and are also described in the example set forth.

In order that this invention be more fully understood, the following preparative and testing examples are set forth. These examples are for the purpose of illustration only and are not to be construed as limiting the scope of the invention in any way.

### EXAMPLES

As used herein, the term "Rt(min)" refers to the HPLC retention time, in minutes, associated with the compound. Unless otherwise indicated, the HPLC method utilized to obtain the reported retention time is as follows:
Column: ACE C8 column, 4.6 x 150 mm
Gradient: 0-100% acetonitrile+methanol 60:40 (20mM Tris phosphate)
Flow rate: 1.5 mL/minute
Detection: 225 nm.

Mass spec. samples were analyzed on a MicroMass Quattro Micro mass spectrometer operated in single MS mode with electrospray ionization. Samples were introduced into the mass spectrometer using chromatography. Mobile phase for all mass spec. analyses consisted of 10mM pH 7 ammonium acetate and a 1:1 acetonitrile-methanol-mixture, column gradient conditions are 5%-100% acetonitrile-methanol over 3.5 mins gradient time and 5 mins run time on an ACE C8 3.0 x 75mm column. Flow rate is 1.2 ml/min.

¹H-NMR spectra were recorded at 400 MHz using a Bruker DPX 400 instrument. The following compounds of formula I were prepared and analyzed as follows.

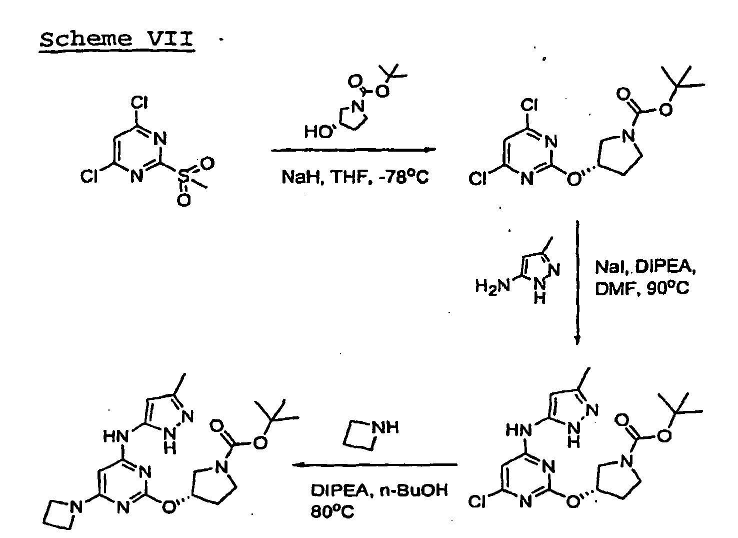

### Example 1

### (S)-3-(4,6-Dichloro-pyrimidin-2-yloxy)-pyrrolidine-1-carboxylic acid tert-butyl ester

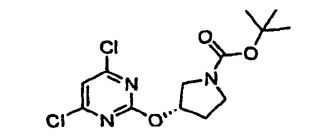

Tetrahydrofuran (40 ml) was carefully added to sodium hydride (60 % in oil, 0.84 g, 21.02 mmol) under nitrogen. (S)-3-Hydroxy-pyrrolidine-1-carboxylic acid *tert-*butyl ester (4.13 g, 22.06 mmol) in tetrahydrofuran (40 ml) was added and the suspension was stirred at room temperature for 2 hrs. The suspension was cooled to -78°C and a solution of 4,6-dichloro-2-methanesulfonyl-pyrimidine (4.55 g, 20.04 mmol) in tetrahydrofuran (40 ml) was added the stirring continued at -78°C for a further 2 h. Sat NH₄Cl and EtOAc was added, the organic layer dried (Na₂SO₄), and concentrated in vacuo. The compound was purified by flash column chromatography (15% EtOAc/hexane) to give the title compound as a white solid (5.27 g, 79 %). 1H NMR (CDCl₃) 1.49 (9H, s), 2.12 - 2.30 (2H, m), 3.55 - 3.71 (4H, m), 5.51 - 5.59 (1H, m), 7.05 (1H, s). MS (ES+): 334.

### Example 2

### (S)-3-[4-Chloro-6-(5-methyl-2H-pyrazol-3-ylamino)-pyrimidin-2-yloxy]-pyrrolidine-1-carboxylic acid tert-butyl ester

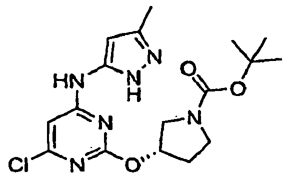

A round bottom flask was charged with of (S)-3-(4,6-dichloro-pyrimidin-2-yloxy)-pyrrolidine-1-carboxylic acid *tert-*butyl ester (5.27 g, 15.83 mmol), 5-methyl-2*H*-pyrazol-3-ylamine (3.07 g, 31.65 mmol), sodium iodide (2.37 g, 15.83 mmol), diisopropylethyl amide (4.99 g, 39.58 mmol, 6.87 ml) and dimethylformamide (60ml) under nitrogen. The reaction mixture was stirred at 90°C for 16h, and then allowed to cool to room temperature. The residue was diluted with ethyl acetate and washed with brine, dried (Na₂SO₄) and concentrated *in vacuo.* The compound was purified by flash chromatography (1:1 hexane:EtOAc) to give the title compound as an off white solid (4.75 g, 78%). 1H NMR (DMSO) 1.50 (9H, d), 2.02 - 2.20 (2H, m), 2.21 (3H, s), 3.32 - 3.45 2H, m), 3.55 - 3.65 (1H, m), 5.40 (1H, d), 6.41 (1H, brs), 7.40 (1H, brs), 10.15 (1H, brs), 12.12 (H, s). MS (ES+) : 395.

### Example 3

### (S)-3-[4-Azetidin-1-yl-6-(5-mothyl-2H-pyrazol-3-ylamino)-pyrimidin-2-yloxy]-pyrrolidine-1-carboxylic acid tert-butyl ester (Compound I-1)

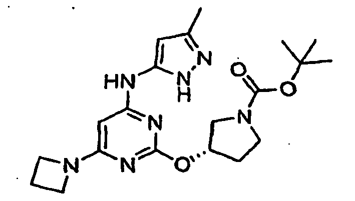

A round bottom flask was charged with (S)-3-[4-chloro-6-(5-methyl-2*H*-pyrazol-3-ylamino)-pyrimidin-2-yloxy]-pyrrolidine-1-carboxylic acid *tert*-butyl ester (200 mg, 0.51 mmol), azetidine (58 mg, 1.02 mmol), diisopropyl ethylamine (162 mg, 1.23 mmol, 0.22 ml) and n-butanol (10ml). The reaction mixture was stirred at 80°C for 14 h and then diluted with ethyl acetate (25ml) and washed with brine. The organic was dried (Na₂SO₄) and concentrated in vacuo to give the title compound as a white solid (180 mg, 85%). 1H NMR (DMSO) 1.40 (9H, d), 2.00 - 2.22 (5H, m), 2.30 - 2.33 (2H, m), 3.51 - 3.57 (1H, m), 3.90 (4H, t), 5.32 (1H, d), 5.86 - 5.93 (2H, brs), 9.11 (1H, s), 11.86 (1H, s). MS (ES+): 416. HPLC Rt (min): 9.058.

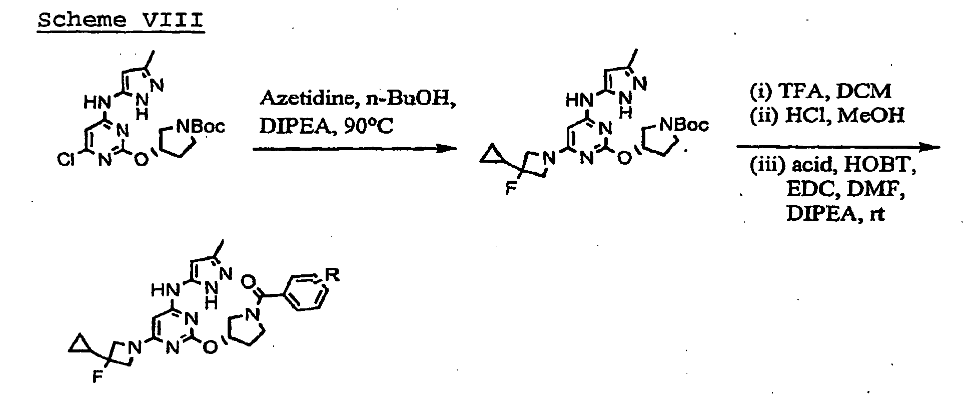

### Example 4

### (S)-tert-butyl 3-(4-(3-cyclopropyl-3-fluoroazetidin-1-yl)-6-(3-methyl-1H-pyrazol-5-ylamino)pyrimidin-2-yloxy)pyrrolidine-1-carboxylate (I-2)

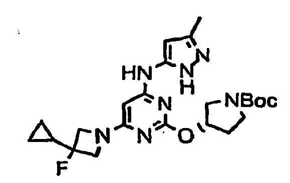

Diisopropylethylamine (1.74 g, 13.52 mmol, 2.40 ml) was added to a suspension of (S)-tert-butyl 3-(4-chloro-6-(3-methyl-1H-pyrazol-5-ylamino)pyrimidin-2-yloxy) pyrrolidine-1-carboxylate (1.00 g, 2.54 mmol) and 3-cyclopropyl-3-fluoroazetidine hydrochloride (768 mg, 5.07 mmol) in n-butanol (20 ml) and the mixture was heated to 90∞C and stirred for 20 hrs. The mixture was allowed to cool and diluted with ethylacetate (80 ml) and water (50 ml). The organic layer was dried (MgS04) and concentrated to give a yellow oil. Purification by flash column chromatography eluting with ethylacetate gave the title compound as an off white solid (130 mg, 11 %). 1H NMR (DMSO, 400 MHz) 0.48 - 0.51 (2H, m), 0.66 - 0.69 (2H, m), 0.88 - 0.93 (1H, m), 1.42 (9H, s), 2.12 - 2.24 (2H, m), 2.27 (3H, s), 3.39 - 3.49 (3H, m), 3.59 - 3.61 (1H, m), 3.89 - 4.02 (4H, m), 5.39 (1H, d), 5.95 (1H, brs), 6.01 (1H, s), 9.24 (1H, s), 11.90 (1H, s). ES+ 474. HPLC Rt (min): 9.668.

### Example 5

### (S)-(5-chloro-2-fluorophenyl) (3-(4-(3-cyclopropyl-3-fluoroazetidin-1-yl)-6-(3-methyl-1H-pyrazol-5-ylamino)pyrimidin-2-yloxy)pyrrolidin-1-yl)methanone (I-3)

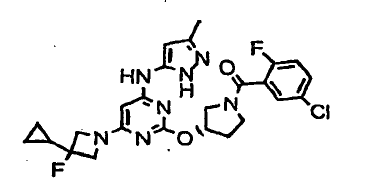

To a solution of (S)-tert-butyl 3-(4-(3-cyclopropyl-3-fluoroazetidin-1-yl)-6-(3-methyl-1H-pyrazol-5-ylamino)pyrimidin-2-yloxy)pyrrolidine-l-carboxylate (105 mg, 0.24 mmol) in dichloromethane (5 ml) was added trifluoroacetic acid (2.5 ml) and the solution stirred at room temperature for lhr. The reaction was concentrated, treated with methanol (5 ml) and concentrated hydrochloric acid (0.5 ml) and concentrated again to give a white solid. The solid was dissolved in DMF (4 ml) and EDC (46 mg, 0.24 mmol), HOBT (32 mg, 0.24 mmol), DIPEA (122 mg, 0.96 mmol, 0.17 ml) and 5-chloro-2-fluorobenzoic acid (42 mg, 0.24 mmol) was added and the solution stirred at room temperature for 40 hrs. The reaction was diluted with sat NaHCO-3 (30 ml) and ethylacetate (30 ml), the organic layer washed with brine, dried (MgSO4) and concentrated to give a yellow oil. Purification by flash column chromatography eluting with 50% EtOAc/hexanes to EtOAc gave the title compound as a pink solid (20 mg, 16 %). 1H NMR (DMSO, 400 MHz) 0.46 - 0.52 (2H, m), 0.65 - 0.68 (2H, m), 1.41 - 1.52 (1H, m), 1.97 - 2.31 (5H, m), 3.39 - 3.51 (1H, m), 3.61 - 3.79 (2H, m), 3.84 - 4.06 (5H, m), 5.39 and 5.49 (0.5H, s), 5.95 (1H, s), 6.03 (1H, s), 7.40 - 7.47 (1H, m), 7.58-7.66 (2H, m), 9.21 and 9.27 (0.5H, s), 11.91 (1H, s). ES+ 530. HPLC Rt (min): 9.226.

### Examples 6: Aurora-2 (Aurora A) Inhibition Assay

Compounds were screened for their ability to inhibit Aurora-2 using a standard coupled enzyme assay (Fox et al., Protein Sci., (1998) 7, 2249). Assays were carried out in-a mixture of 100mM Hepes (pH7.5), 10mM MgCl₂, 1mM DTT, 25mM NaCl, 2.5mM phosphoenolpyruvate, 300 us NADH, 30 µg/ml pyruvate kinase and 10 µg/ml lactate dehydrogenase. Final substrate concentrations in the assay are 400µM ATP (Sigma Chemicals) and 570µM peptide (Kemptide, American Peptide, Sunnyvale, CA). Assays were carried out at 30 °C and in the presence of 40nM Aurora-2.

An assay stock buffer solution was prepared containing all of the reagents listed above, with the exception of Aurora-2 and the test compound of interest. 55 µl of the stock solution was placed in a 96 well plate followed by addition of 2 µl of DMSO stock containing serial dilutions of the test compound (typically starting from a final concentration of 7.5µM). The plate was preincubated for 10 minutes at 30°C and the reaction initiated by addition of 10 µl of Aurora-2. Initial reaction rates were determined with a Molecular Devices SpectraMax Plus plate reader over a 10 minute time course. IC50 and Ki data were calculated from non-linear regression analysis using the Prism software package (GraphPad Prism version 3.0cx for Macintosh, GraphPad Software, San Diego California, USA).

Compound I-1 was found to have a Ki value < 0.050 uM. Compounds I-2 and I-3 were found to have a Ki value < 0.010 uM.

### Example 7: Aurora-1 (Aurora B) Inhibition Assay (radiometric)

An assay buffer solution was prepared which consisted of 25 mM HEPES (pH 7.5), 10 mM MgCl₂, 0.1% BSA and 10% glycerol. A 22 nM Aurora-B solution, also containing 1.7 mM DTT and 1.5 mM Kemptide (LRRASLG), was prepared in assay buffer. To 22 µL of the Aurora-B solution, in a 96-well plate, was added 2 µl of a compound stock solution in DMSO and the mixture allowed to equilibrate for 10 minutes at 25°C. The enzyme reaction was initiated by the addition of 16 µl stock [γ-³³P]-ATP solution (~20 nCi/µL) prepared in assay buffer, to a final assay concentration of 800 µM. The reaction was stopped after 3 hours by the addition of 16 µL 500 mM phosphoric acid and the levels of ³³P incorporation into the peptide substrate were determined by the following method.

A phosphocellulose 96-well plate (Millipore, Cat no. MAPHNOB50) was pre-treated with 100 µL of a 100 mM phosphoric acid prior to the addition of the enzyme reaction mixture (40 µL). The solution was left to soak on to the phosphocellulose membrane for 30 minutes and the plate subsequently washed four times with 200 µL of a 100 mM phosphoric acid. To each well of the dry plate was added 30 µL of Optiphase 'SuperMix' liquid scintillation cocktail (Perkin Elmer) prior to scintillation counting (1450 Microbeta Liquid Scintillation Counter, Wallac). Levels of non-enzyme catalyzed background radioactivity were determined by adding 16 µL of the 500 mM phosphoric acid to control wells, containing all assay components (which acts to denature the enzyme), prior to the addition of the [γ-³³P]-ATP solution. Levels of enzyme catalyzed ³³P incorporation were calculated by subtracting mean background counts from those measured at each inhibitor concentration. For each Ki determination 8 data points, typically covering the concentration range 0 - 10 µM compound, were obtained in duplicate (DMSO stocks were prepared from an initial compound stock of 10 mM with subsequent 1:2.5 serial dilutions). Ki values were calculated from initial rate data by non-linear regression using the Prism software package (Prism 3.0, Graphpad Software, San Diego, CA).

Compounds I-1 and I-2 were found to have a Ki value <0.50 uM. Compound I-3 was found to have a Ki value < 0.025 uM.

### Example 8: Itk Inhibition Assay

The compounds of the present invention were evaluated as inhibitors of human Itk kinase using a radioactivity-based assay.

Assays were carried out in a mixture of 20 mM MOPS (pH 7.0), 10mM MgCl₂, 0.1% BSA and 1mM DTT. Final substrate concentrations in the assay were 7.5 µM [γ-³³P] ATP (400µCi ³³P ATP/ µmol ATP, Amersham Pharmacia Biotech / Sigma Chemicals) and 3µM peptide (SAM68 protein Δ332-443). Assays were carried out at 25 °C. in the presence of 50 nM Itk. An assay stock buffer solution was prepared containing all of the reagents listed above, with the exception of ATP and the test compound of interest. 50µL of the stock solution was placed in a 96 well plate followed by addition of 2µL of DMSO stock containing serial dilutions of the test compound (typically starting from a final concentration of 50µM with 2-fold serial dilutions) in duplicate (final DMSO concentration 2%). The plate was pre-incubated for 10 minutes at 25°C and the reaction initiated by addition of 50µL [γ-³³P]ATP (final concentration 7.5µM).

The reaction was stopped after 10 minutes by the addition of 100µL 0.2M phosphoric acid + 0.01% TWEEN 20. A multiscreen phosphocellulose filter 96-well plate (Millipore, Cat no. MAPHN0B50) was pretreated with 100µL 0.2M phosphoric acid + 0.01% TWEEN 20 prior to the addition of 170µL of the stopped assay mixture. The plate was washed with 4 x 200µL 0.2M phosphoric acid + 0.01% TWEEN 20. After drying, 30µL Optiphase 'SuperMix' liquid scintillation cocktail (Perkin Elmer) was added to the well prior to scintillation counting (1450 Microbeta Liquid Scintillation Counter, Wallac).

Ki(app) data were calculated from non-linear regression analysis of the initial rate data using the Prism software package (GraphPad Prism version 3.0cx for Macintosh, GraphPad Software, San Diego California, USA).

Compound I-1 was found to have a Ki value < 1 uM.

### Example 9: JAK3 Inhibition Assay

Compounds were screened for their ability to inhibit JAK using the assay shown below. Reactions were carried out in a kinase buffer containing 100 mM HEPES (pH 7.4), 1 mM DTT, 10 mM MgCl₂, 25 mM NaCl, and 0.01% BSA.
Substrate concentrations in the assay were 5 µM ATP (200 uCi/µmole ATP) and 1 µM poly(Glu)₄Tyr. Reactions were carried out at 25 °C and 1 nM JAK3.

To each well of a 96 well polycarbonate plate was added 1.5 µl of a candidate JAK3 inhibitor along with 50 µl of kinase buffer containing 2 µM poly(Glu) 9Tyr and 10 µM ATP. This was then mixed and 50µl of kinase buffer containing 2 nM JAK3 enzyme was added to start the reaction. After 20 minutes at room temperature (25C), the reaction was stopped with 50µl of 20% trichloroacetic-acid (TCA) that also contained 0.4 mM ATP. The entire contents of each well were then transferred to a 96 well glass fiber filter plate using a TomTek Cell Harvester. After washing, 60 µl of scintillation fluid was added and ³³P incorporation detected on a Perkin Elmer TopCount.

Compounds I-1 and I-2 were found to have a Ki value < 0.50 uM. Compound I-3 was found to have a Ki value < 0.25 uM.

### Example 10: JACK2 Inhibition Assay

The assays are as described above in Example 3 except that JAK-2 enzyme is used, the final poly (Glu)₄Tyr concentration is 15 µM. and final ATP concentration is 12 µM.

Compound I-1 was found to have a Ki value < 0.25 uM. Compounds I-2 and I-3 were found to have a Ki value < 0.10 uM.

### Examples 11: FLT-3 Inhibition Assay

Compounds can be screened for their ability to inhibit FLT-3 activity using a radiometric filter-binding assay. This assay monitors the 33P incorporation into a substrate poly(Glu, Tyr) 4:1 (pE4Y). Reactions are carried out in a solution containing 100 mM HEPES (pH 7.5), 10 mM MgCl₂, 25 mM NaCl, 1 mM DTT, 0.01% BSA and 2.5% DMSO. Final substrate concentrations in the assay are 90 µM ATP and 0.5mg/ml pE4Y (both from Sigma Chemicals, St Louis, MO). The final concentration of a compound of the present invention is generally between 0.01 and 5 µM. Typically, a 12-point titration is conducted by preparing serial dilutions from 10 mM DMSO stock of test compound. Reactions are carried out at room temperature.

Two assay solutions are prepared. Solution 1 contains 100 mM HEPES (pH 7.5), 10 mM MgCl₂, 25 mM NaCl, 1 mg/ml pE4Y and 180 mM ATP(containing 0.3mCi of [γ-33^{P}]ATP for each reaction). Solution 2 contains 100 mM HEPES (pH 7.5), 10 mM MgCl₂, 25 mM NaCl, 2 mM DTT, 0.02% BSA and 3 nM FLT-3.
The assay is run on a 96 well plate by mixing 50µl each of Solution 1 and 2.5 ml of the compounds of the present invention. The reaction is initiated with Solution 2. After incubation for 20 minutes at room temperature, the reaction is stopped with 50µl of 20% TCA containing 0.4mM of ATP. All of the reaction volume is then transferred to a filter plate and washed with 5% TCA by a Harvester 9600 from TOMTEC (Hamden, CT). The amount of ³³P incorporation into pE4y is analyzed by a Packard Top Count Microplate Scintillation Counter (Meriden, CT). The data is fitted using Prism software to get an IC50 or Ki.

While we have described a number of embodiments of this invention, it is apparent that our basic examples may be altered to provide other embodiments that utilize or encompass the compounds, methods, and processes of this invention. Therefore, it will be appreciated that the scope of this invention is to be defined by the appended claims.

## Claims

1. A compound of formula I: 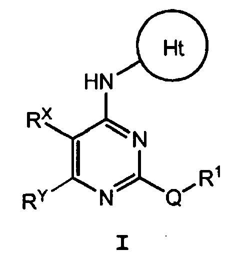 or a pharmaceutically acceptable salt thereof, wherein:
Ht is thiazole or pyrazole; Ht is optionally and independently substituted with R² and R^{2'}; in particular Ht is 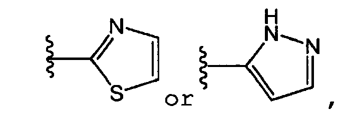 wherein Ht is optionally and independently substituted with R² and R^{2'};
Q is -O-, -NR'-, -S-, -C(R')₂-, or -(C=O)-;
R^{X} is H, C₁₋₆aliphatic, NO₂, CN, halo, NH₂, N(C₁₋₄aliphatic), N(C₁₋₄aliphatic)₂, O(C₁₋₄aliphatic), OH, or -N(C=O) (C₁₋₄aliphatic); wherein said aliphatic is optionally substituted with 1-3 fluoro;
R^{Y} is -Z-R¹⁰;
R¹ is T- (Ring D);
Ring D is a 4-7 membered monocyclic heterocyclyl or a carbocyclyl ring, wherein said heterocyclyl has 1-4 ring heteroatoms selected from O, N, and S; Ring D is optionally fused to Ring D';
Ring D' is a 5-8 membered partially saturated or fully unsaturated monocyclic ring containing 0-4 ring heteroatoms selected from nitrogen, oxygen or sulfur;
Ring D and Ring D' are each independently and optionally substituted with 0-4 occurrences of oxo or -Z-R⁵;
each T is independently a C₁₋₄ alkylidene chain or is absent;
R² and R^{2'} are independently -R, -W-R⁶, or R⁸; or R² and R^{2'} are taken together with their intervening atoms to form a fused, 5-8 membered, unsaturated or partially unsaturated, ring having 0-3 ring heteroatoms selected from nitrogen, oxygen, or sulfur; wherein the 5-8 membered ring is independently substituted with halo, oxo, -CN, -NO₂, -R⁷, or -Y-R⁶;
each Z, Y, and W is independently a bond or a C₁₋₁₀ alkylidene chain wherein up to six methylene units of the alkylidene chain are optionally replaced by V;
each V is selected from -O-, -C(=O)-, -S(O)-, -S(O)₂-, -S-, or -N(R⁴)-;
each R³ and R⁵ is independently -R, -halo, -OR, -C(=O)R, -CO₂R, -COCOR, COCH₂COR, -NO₂, -CN, -S(O)R, -S(O)₂R, -SR, -N(R⁴)₂, -CON(R⁷)₂, -SO₂N (R⁷)₂, -OC(=O)R, -N(R⁷) COR, -N(R⁷) CO₂(C₁₋₆ aliphatic), -N(R⁴)N(R⁴)₂, -C=NN(R')₂, -C=N-OR, -N(R⁷)CON(R⁷)₂, -N(R⁷)SO₂N(R⁷)₂, -N(R⁴)SO₂R, or -OC(=O)N(R⁷)₂;
each R is hydrogen, a C₁₋₆aliphatic group, a C₆₋₁₀aryl ring, a heteroaryl ring having 5-10 ring atoms, or a heterocyclyl ring having 4-10 ring atoms; wherein said heteroaryl or heterocyclyl ring has 1-4 ring heteroatoms selected from nitrogen, oxygen, or sulfur; R is optionally substituted with 0-6 R⁹;
each R⁴ is -R⁷, -COR⁷, -CO₂R⁷, -CON(R⁷)₂, or -SO₂R⁷;
each R⁶ is independently hydrogen or an optionally substituted C₁₋₆ aliphatic group; or two R⁶ groups on the same nitrogen atom are taken together with the nitrogen atom to form an optionally substituted 4-6 membered heterocyclyl or heteroaryl ring;
each R⁷ is independently hydrogen or an optionally substituted C₁₋₆ aliphatic group; or two R⁷ on the same nitrogen are taken together with the nitrogen to form an optionally substituted 4-8 membered heterocyclyl or heteroaryl ring containing 1-4 heteroatoms selected from nitrogen, oxygen, or sulfur;
each R⁸ is halogen, -CN, or -NO₂;
each R⁹ is -R', -halo, -OR', -C(=O)R', -CO₂R', -COCOR', COCH₂COR', -NO₂, -CN, -S(O)R', -S(O)₂R', -SR', -N(R')₂, -CON(R')₂, -SO₂N(R')₂, -OC(=O)R', -N(R')COR', -N(R')CO₂(C₁₋₆ aliphatic), -N(R')N(R')₂, -N(R')CON(R')₂, -N(R')SO₂N(R')₂, -N(R')SO₂R', -OC(=O)N(R')₂, =NN(R')₂, =N-OR', or =O;
each R¹⁰ is a 4-membered heterocyclic ring containing 1 heteroatom selected from O, NR¹¹, and S; each R¹⁰ is optionally substituted with 0-6 occurrences of J;
each J is independently R, -halo, -OR, oxo, -C(=O)R, -CO₂R, -COCOR, -COCH₂COR, -NO₂, -CN, -S(O)R, -S(O)₂R, -SR, -N(R⁴)₂, -CON(R⁷)₂, -SO₂N(R⁷)₂, -OC(=O)R, -N(R⁷)COR, -N(R⁷)CO₂(C₁₋₆ aliphatic), -N(R⁴)N(R⁴)₂, =NN(R⁴)₂, =N-OR, -N(R⁷)CON(R⁷)₂, -N(R⁷)SO₂N(R⁷)₂, -N(R⁴)SO₂R, -OC(=O)N(R⁷)₂, or -OP(=O) (OR")₂; or
2 J groups, on the same atom or on different atoms, together with the atom(s) to which they are bound, form a 3-8 membered saturated, partially saturated, or unsaturated ring having 0-2 heteroatoms selected from O, N, or S; wherein 1-4 hydrogen atoms on the ring formed by the 2 J groups is optionally replaced with halo, C₁₋₃alkyl, or -O(C₁₋₃alkyl); or two hydrogen atoms on the ring are optionally replaced with oxo or a spiro-attached C₃₋₄ cycloalkyl; wherein said C₁₋₃alkyl is optionally substituted with 1-3 fluorine;
each R¹¹ is -R⁷, -COR⁷, -CO₂R⁷, -CON(R⁷)₂, or -SO₂R⁷;
each R' is independently hydrogen or a C₁₋₆ aliphatic group optionally substituted with 0-4 occurrences of NH₂, NH(C₁₋₄aliphatic) , N(C₁₋₄aliphatic)₂, halogen, C₁₋₄aliphatic, OH, O(C₁₋₄aliphatic), NO₂, CN, CO₂H, CO₂(C₁₋₄aliphatic), O(haloC₁₋₄ aliphatic), or haloC₁₋₄aliphatic; or, two R', together with the atom(s) to which they are attached, form an optionally substituted 3-6 membered carbocyclyl or heterocyclyl; and
each R" is independently H or C₁₋₂alkyl;
wherein an aryl (including aralkyl, aralkoxy, aryloxyalkyl) or heteroaryl (including heteroaralkyl and heteroarylalkoxy) group may contain one or more substituents and thus may be "optionally substituted";
wherein unless otherwise defined above and herein, suitable substituents on the unsaturated carbon atom of an aryl or heteroaryl group are selected from halogen; -R^{o}; -OR^{o}; -SR°; phenyl (Ph) optionally substituted with R^{o}; -O(Ph) optionally substituted with R^{o}; -(CH₂)₁₋₂(Ph), optionally substituted with R^{o}; -CH=CH(Ph), optionally substituted with R^{o}; a 4-6 membered heteroaryl or heterocyclic ring optionally substituted with R^{o}; -NO₂; -CN; -N(R^{o})₂; -NR^{o}C(O)R^{o}; -NR^{o}C(S)R^{o}; -NR^{o}C(O)N(R^{o})₂; -NR^{o}C (S)N (R^{o})₂; -NR^{o}CO₂R^{o}; -NR^{o}NR^{o}-, -NR^{o}NR^{o}C (O) R^{o}; -NR^{o}NR^{o}C(O)N(R^{o})₂; -NR^{o}NR^{o}CO₂R^{o}; -C(O)C(O)R^{o}; -C(O)CH₂C(O)R^{o}; -CO₂R^{o}; -C(O)R°; -C(S)R^{o}; -C(O)N(R^{o})₂; -C(S)N(R^{o})₂; -OC(O)N(R^{o})₂; -OC(O)R^{o}; -C(O)N(OR^{o})R^{o}; -C(NOR^{o})R^{o}; -S(O)₂R^{o}; -S(O)₃R^{o}; -SO₂N(R^{o})₂; -S(O)R^{o}; -NR^{o}SO₂N(R^{o})₂; -NR^{o}SO₂R^{o}; -N(OR^{o})R^{o}; -C(=NH)-N(R^{o})₂; -C(=NH)-OR^{o}; -P(O)₂R^{o}; -PO(R^{o})₂; -OPO(R^{o})₂; or -(CH₂)₀₋₂NHC(O)R^{o}; wherein each independent occurrence of R^{o} is selected from hydrogen, optionally substituted C₁₋₆ aliphatic, an unsubstituted 4-6 membered heteroaryl or heterocyclic ring, phenyl, -O(Ph), or -CH₂(Ph), or, notwithstanding the definition above, two independent occurrences of R^{o}, on the same substituent or different substituents, taken together with the atom(s) to which each R^{o} group is bound, to form an optionally substituted 3-12 membered saturated, partially unsaturated, or fully unsaturated monocyclic or bicyclic ring having 0-4 heteroatoms independently selected from nitrogen, oxygen, or sulfur and
wherein optional substituents on the aliphatic group of R^{o} are selected from NH₂, NH(C₁₋₄aliphatic) , N(C₁₋₄aliphatic)₂, halogen, C₁₋₄aliphatic, OH, O(C₁₋₄aliphatic) , NO₂, CN, CO₂H, CO₂(C₁₋₄aliphatic) , O(haloC₁₋₄ aliphatic), or haloC₁₋₄aliphatic, wherein each of the foregoing C₁₋₄aliphatic groups of R^{o} is unsubstituted.

2. The compound of claim 1, wherein Ring D is an optionally substituted 4-7 membered monocyclic ring selected from a heterocyclyl ring, in particular an optionally substituted piperidinyl or pyrrolidinyl ring or a carbocyclyl ring, wherein said heterocyclic ring has 1-2 heteroatoms selected from O, N, and S.

3. The compound of claim 1 or 2, wherein R is H or optionally substituted C₁₋₆ aliphatic.

4. The compound of any one of claims 1-3, wherein Z is absent.

5. The compound of any one of claims 1-4, wherein Z is a C₁₋₆ alkylidene chain, in particular a C₁₋₄ alkylidene chain, wherein 1-2 methylene units of Z is optionally replaced by O, -N(R⁶)-, or S.

6. The compound of any one of claim 1 to 5, wherein R¹⁰ is an optionally substituted azetidine, in particular R¹⁰ is represented by formula i:

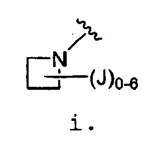

7. The compound of any one of claims 1-5, wherein R^{Y} is represented by formula ii-a':

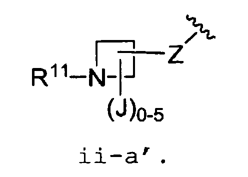

8. The compound of any one of claims 1-7 as represented by formula Ia:

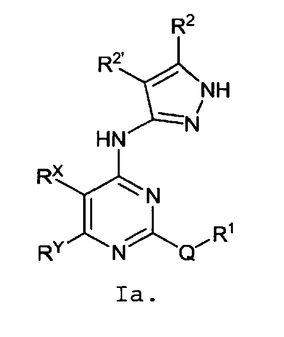

9. The compound of any one of claims 1-7 as represented by formula Ib:

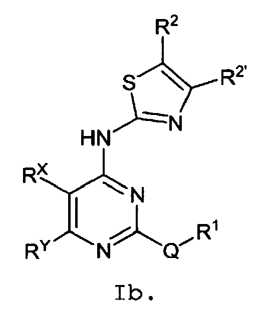

10. The compound of claim 8 or 9, wherein R^{2'} is H or optionally substituted C₁₋₃ aliphatic.

11. The compound of any one of claims 8-10, wherein R² is H or optionally substituted C₁₋₃ aliphatic.

12. The compound of any one of claims 8-10, wherein R⁵ is an optionally substituted group selected from C₁₋₆aliphatic or phenyl.

13. The following compounds:

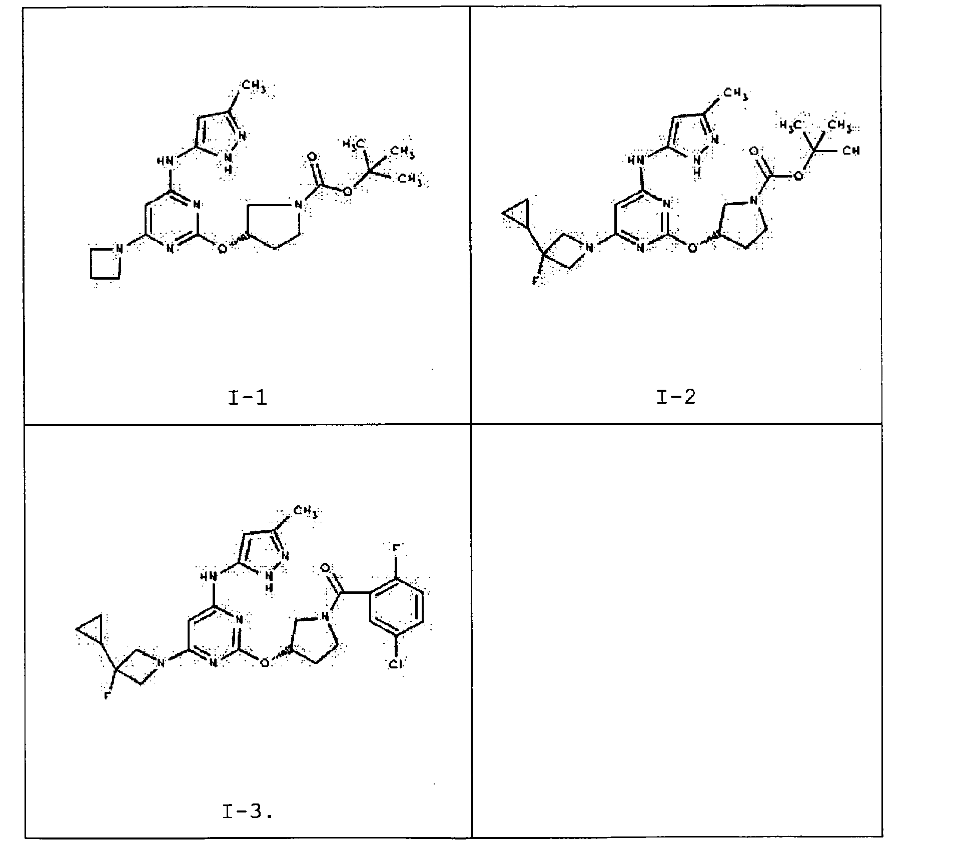

14. A composition comprising a compound of any one of claims 1-13 and a pharmaceutically acceptable carrier, adjuvant, or vehicle.

15. A method of inhibiting Aurora protein kinase activity in a biological sample comprising contacting said biological sample with a compound of any one of claims 1-13.

16. The use of a compound of any one of claims 1-13 in the manufacture of a medicament for treating a proliferative disorder in a patient, in particular a proliferative disorder selected from melanoma, myeloma, leukemia, lymphoma, neuroblastoma, or a cancer selected from colon, breast, gastric, ovarian, cervical, lung, central nervous system (CNS), renal, prostate, bladder, pancreatic, brain (gliomas), head and neck, kidney, liver, melanoma, sarcoma, or thyroid cancer.

17. The use of a compound of any one of claims 1-13 or a pharmaceutically acceptable salt thereof, and another therapeutic agent, in particular taxanes, inhibitors of bcr-abl, inhibitors of EGFR, DNA damaging agents, and/or antimetabolites or Paclitaxel, Gleevec, dasatinib, nilotinib, Tarceva, Iressa, cisplatin, oxaliplatin, carboplatin, anthracyclines, AraC and/or 5-FU and/or camptothecin, doxorubicin, idarubicin, Cisplatin, taxol, taxotere, vincristine, tarceva, the MEK inhibitor, U0126, a KSP inhibitor, vorinostat, Gleevec, dasatinib, and/or nilotinib in the manufacture of a medicament for treating cancer in a subject in need thereof.

## Patentansprüche

1. Verbindung mit der Formel I: 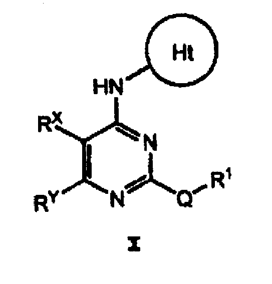 oder ein pharmazeutisch verträgliches Salz davon, wobei:
- Ht Thiazol oder Pyrazol bedeutet, Ht wahlweise und unabhängig voneinander mit R² und R^{2'} substituiert ist
und Ht insbesondere 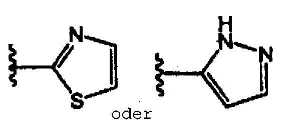 bedeutet, worin Ht wahlweise und unabhängig voneinander mit R² und R^{2'} substituiert ist,
- Q -O-, -NR'-, -S-, -C(R')₂- oder -(C=O)-,
- R^{X} H, C₁- bis C₆-aliphatisch, NO₂, CN, Halogen, NH₂, N(C₁- bis C₄-aliphatisch), N(C₁- bis C₄-aliphatisch)₂, O(C₁- bis C₄-aliphatisch), OH oder -N(C=O) (C₁- bis C₄-aliphatisch), wobei aliphatisch wahlweise mit 1 bis 3 Fluoratomen substituiert ist,
- R^{Y} -Z-R¹⁰ ,
- R¹ T- (Ring D),
- Ring D einen 4- bis 7gliedrigen monocyclischen Heterocyclyl- oder Carbocyclylring, wobei der Heterocyclylring 1 bis 4 Ringheteroatome besitzt, die aus O, N und S ausgewählt sind, und Ring D wahlweise mit Ring D' anelliert ist, und
- Ring D' einen 5- bis 8gliedrigen teilweise gesättigten oder vollständig ungesättigten monocyclischen Ring, der 0 bis 4 Ringheteroatome enthält, die aus Stickstoff, Sauerstoff oder Schwefel ausgewählt sind, bedeutet,
- Ring D und Ring D' jeweils unabhängig voneinander und wahlweise mit 0 bis 4 Vorkommen von Oxo oder -Z-R⁵ substituiert sind,
- jeder T unabhängig voneinander eine C₁- bis C₄-Alkylidenkette bedeutet oder fehlt,
- R² und R^{2'} jeweils unabhängig voneinander -R, -W-R⁶ oder R⁸ bedeuten oder R² und R^{2'} mit ihren Zwischenatomen zusammengenommen werden, um einen anellierten 5- bis 8gliedrigen ungesättigten oder teilweise ungesättigten Ring mit 0 bis 3 Ringheteroatomen, die aus Stickstoff, Sauerstoff oder Schwefel ausgewählt sind, zu bilden, wobei der 5- bis 8gliedrige Ring unabhängig voneinander mit Halogen, Oxo, -CN, -NO₂, -R⁷ oder -Y-R⁶ substituiert ist,
- jeder Z, Y und W unabhängig voneinander eine Bindung oder eine C₁- bis C₁₀-Alkylidenkette, worin bis zu sechs Methyleneinheiten der Alkylidenkette wahlweise durch V ersetzt sind, bedeutet,
- jeder V aus -O-, -C(=O)-, -S(O)-, -S(O)₂-, -S- oder -N(R₄)- ausgewählt ist,
- jeder R³ und R⁵ unabhängig voneinander -R, -Halogen, -OR, -C(=O)R, -CO₂R, -COCOR, COCH₂COR, -NO₂, -CN, -S(O)R, -S(O)₂R, -SR, -N(R⁴)₂, -CON(R⁷)₂, -SO₂N(R⁷)₂, -OC(=O)R, -N(R⁷)COR, -N(R⁷)CO₂ (C₁- bis C₆-aliphatisch), -N(R⁴)N(R⁴)₂, -C=NN(R⁴)₂, -C=N-OR, -N(R⁷)CON(R⁷)₂, -N (R⁷) SO₂N (R⁷)₂, -N (R⁴) SO₂R oder -OC (=O) N (R⁷)₂,
- jeder R Wasserstoff, eine C₁- bis C₆-aliphatische Gruppe, einen C₆- bis C₁₀-Arylring, einen Heteroarylring mit 5 bis 10 Ringatomen oder einen Heterocyclylring mit 4 bis 10 Ringatomen, wobei dieser Heteroaryl- oder Heterocyclylring 1 bis 4 Ringheteroatome, die aus Stickstoff, Sauerstoff oder Schwefel ausgewählt sind, besitzt und R wahlweise mit 0 bis 6 R⁹ substituiert ist,
- jeder R⁹ -R⁷, -COR⁷, -CO₂R⁷, -CON(R⁷)₂ oder -SO₂R⁷ und
- jeder R⁶ unabhängig voneinander Wasserstoff oder eine wahlweise substituierte C₁- bis C₆-aliphatische Gruppe bedeutet oder zwei R⁶-Gruppen an demselben Stickstoffatom mit dem Stickstoffatom zusammengenommen werden, um einen wahlweise substituierten 4- bis 6gliedrigen Heterocyclyl- oder Heteroarylring zu bilden,
- jeder R⁷ unabhängig voneinander Wasserstoff oder eine wahlweise substituierte C₁- bis C₆-aliphatische Gruppe bedeutet oder zwei R⁷ an demselben Stickstoffatom mit dem Stickstoffatom zusammengenommen werden, um einen wahlweise substituierten 4- bis 8gliedrigen Heterocyclyl- oder Heteroarylring zu bilden, der 1 bis 4 Heteroatome, die aus Stickstoff, Sauerstoff oder Schwefel ausgewählt sind, enthält,
- jeder R⁸ Halogen, -CN oder -NO₂,
- jeder R⁹ -R', -Halogen, -OR', -C(=O)R', -CO₂R', -COCOR', COCH₂COR', -NO₂, -CN, -S(O)R', -S(O)₂R', -SR', -N(R')₂, -CON(R')₂, -SO₂N(R')₂, -OC(=O)R', -N(R')COR', -N(R')CO₂(C₁- bis C₆-aliphatisch), -N(R')N(R')₂, -N(R')CON(R')₂, -N(R')SO₂N(R')₂, -N(R')SO₂R', -OC(=O)N(R')₂, =NN(R')₂, =N-OR' oder =O,
- jeder R¹⁰ einen 4gliedrigen heterocyclischen Ring, der 1 Heteroatom, das aus O, NR¹¹ und S ausgewählt ist, enthält, wobei jeder R¹⁰ wahlweise mit 0 bis 6 Vorkommen von J substituiert ist, und
- jeder J unabhängig voneinander R, -Halogen, -OR, Oxo, -C(=O)R, -CO₂R, -COCOR, -COCH₂COR, -NO₂, -CN, -S(O)R, -S(O)₂R, -SR, -N(R⁴)₂, -CON(R⁷)₂, -SO₂N(R⁷)₂, -OC(=O)R, -N(R⁷)COR, -N(R⁷)CO₂(C₁- bis C₆-aliphatisch), -N(R⁴)N(R⁴)₂, =NN(R⁴)₂, =N-OR, -N(R⁷)CON(R⁷)₂, -N(R⁷) SO₂N (R')₂, -N (R⁴) SO₂R, -OC (=O) N (R⁷)₂ oder -OP(=O)(OR")₂ bedeutet oder
- 2 J-Gruppen an demselben Atom oder verschiedenen Atomen zusammen mit dem/den Atom/en, an welche/s sie gebunden sind, einen 3- bis 8gliedrigen gesättigten, teilweise gesättigten oder ungesättigten Ring mit 0 bis 2 Heteroatomen, die aus O, N oder S ausgewählt sind, bilden, wobei 1 bis 4 Wasserstoffatome an dem von den 2 J-Gruppen gebildeten Ring wahlweise durch Halogen, C₁- bis C₃-Alkyl oder -O(C₁- bis C₃-Alkyl) oder zwei Wasserstoffatome an dem Ring wahlweise durch Oxo oder spiro-gebundenes C₃- bzw. C₄-Cycloalkyl ersetzt sind, wobei dieses C₁- bis C₃-Alkyl wahlweise mit 1 bis 3 Fluoratomen substituiert ist,
- jeder R¹¹ -R⁷, -COR⁷, -CO₂R⁷, -CON(R⁷)₂ oder -SO₂R⁷ und
- jeder R' unabhängig voneinander Wasserstoff oder eine C₁- bis C₆-aliphatische Gruppe, die wahlweise mit 0 bis 4 Vorkommen von NH₂ substituiert ist, NH(C₁- bis C₄-aliphatisch), N(C₁- bis C₄-aliphatisch)₂, Halogen, C₁- bis C₄-aliphatisch, OH, O(C₁- bis C₄-aliphatisch), NO₂, CN, CO₂H, CO₂(C₁- bis C₄-aliphatisch), O(HalogenC₁- bis C₄-aliphatisch) oder HalogenC₁- bis C₄-aliphatisch bedeutet oder zwei R' zusammen mit dem/den Atom/en, an welche/s sie gebunden sind, ein wahlweise substituiertes 3- bis 6gliedriges Carbocyclyl- oder Heterocyclyl bilden und jeder R" unabhängig voneinander H oder C₁- bzw. C₂-Alkyl bedeutet;
- wobei eine Aryl (einschließlich Aralkyl, Aralkoxy, Aryloxyalkyl) oder Heteroaryl (einschließlich Heteroaralkyl und Heteroarylalkoxy) Gruppe einen oder mehrere Substituenten aufweisen können und demnach "wahlweise substituiert" sein kann;
- wobei geeignete Substituenten an dem ungesättigten Kohlenstoffatom einer Aryl- oder Heteroarylgruppe, sofern sie nicht oben und hierin anders definiert sind ausgewählt sind aus Halogen; -R^{o}; -OR^{o}; -SR^{o}; Phenyl (Ph), gegebenenfalls substituiert mit R^{o}; -O(Ph) gegebenenfalls substituiert mit R^{o}; -(CH₂)₁₋₂(Ph), gegebenenfalls substituiert mit R^{o}; -CH=CH(Ph), gegebenenfalls substituiert mit R^{o}; ein 4-6 gliedriger Heteroaryl oder heterozyklischer Ring, gegebenenfalls substituiert mit R^{o}; -NO₂; -CN; -N(R^{o})₂; -NR^{o}C(O)R^{o}; -NR^{o}C(S)R^{o}; -NR^{o}C(O)N(R^{o})₂; -NR^{o}C(S)N(R^{o})₂; -NR^{o}CO₂R^{o}; -NR^{o}NR^{o}-, -NR^{o}NR^{o}C (O) R^{o}; -NR^{o}NR^{o}C (O) N (R^{o}) ₂; -NR^{o}NR^{o}CO₂R^{o}; -C(O)C(O)R^{o}; -C(O)CH₂C(O)R^{o}; -CO₂R^{o}; -C(O)R^{o}; -C(S)R^{o}; -C(O)N(R^{o})₂; -C(S)N(R^{o})₂; -OC(O)N(R^{o})₂; -OC(O)R^{o}; -C(O)N(OR^{o})R^{o}; -C(NOR^{o})R^{o}; -S(O)₂R^{o}; -S(O)₃R^{o}; -SO₂N(R^{o})₂; -S(O)R^{o}; -NR^{o}SO₂N(R^{o})₂; -NR^{o}SO₂R^{o}; -N(OR^{o})R^{o}; -C(=NH)-N(R^{o})₂; -C(=NH)-OR^{o}; -P(O)₂R^{o}; -PO(R^{o})₂; -OPO(R^{o})₂; oder -(CH₂)₀₋₂NHC(O)R^{o}; wobei jedes R^{o} unabhängig voneinander ausgewählt ist aus Wasserstoff (H) gegebenfalls substituiertem C₁₋₆ aliphatisch, einem unsubstituiertem 4-6 gliedrigen Heteroaryl oder heterozyklischen Ring, Phenyl, -O(Ph), oder -CH₂ (Ph), oder abweichend von der obigen Definition zwei unabhängig voneinander auftretende R^{o}, am gleichen Sustituenten oder an verschiedenen Substituenten mit dem Atom/den Atomen an die jede R^{o}-Gruppe gebunden ist, zusammengenommen sind, um einen gegebenenfalls substituierten 3-12 gliedrigen gesättigten, teilweise ungesättigten, oder vollständig ungesättigten monozyklischen oder bizyklischen Ring mit 0-4 Heteroatomen, unabhängig voneinander ausgewählt aus Stickstoff, Sauerstoff oder Schwefel zu bilden und
- worin optionale Substituenten an der aliphatischen Gruppe von R^{o} ausgewählt sind aus NH₂, NH (C₁₋₄aliphatisch), N(C₁₋₄aliphatisch)₂, halogen, C₁₋₄aliphatisch, OH, O (C₁₋₄aliphatisch), NO₂, CN, CO₂H, CO₂ (C₁₋₄aliphatisch), O (haloC₁₋₄ aliphatisch), oder haloC₁₋₄aliphatisch, wobei jede der vorangehenden C₁₋₄aliphatischen Gruppen von R^{o} unsubstituiert ist.

2. Verbindung nach Anspruch 1, wobei Ring D einen wahlweise substituierten 4- bis 7gliedrigen monocyclischen Ring bedeutet, der aus einem Heterocyclylring, insbesondere einem wahlweise substituierten Piperidinyl- bzw. Pyrrolidinylring, oder einem Carbocyclylring ausgewählt ist, wobei der heterocyclische Ring 1 bzw. 2 Heteroatome, die aus O, N und S ausgewählt sind, besitzt.

3. Verbindung nach Anspruch 1 oder 2, wobei R² H oder wahlweise substituiertes C₁- bis C₆-aliphatisch bedeutet.

4. Verbindung nach einem der Ansprüche 1 bis 3, wobei Z fehlt.

5. Verbindung nach einem der Ansprüche 1 bis 4, wobei Z eine C₁- bis C₆-Alkylidenkette, insbesondere eine C₁- bis C₄-Alkylidenkette, worin 1 bzw. 2 Methyleneinheiten von Z wahlweise durch O, -N(R⁶)- oder S ersetzt sind, bedeutet.

6. Verbindung nach einem der Ansprüche 1 bis 5, wobei R¹⁰ wahlweise substituiertes Azetidin bedeutet und insbesondere repräsentiert wird von der Formel i:

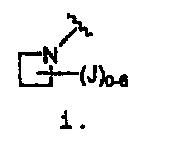

7. Verbindung nach einem der Ansprüche 1 bis 5, wobei R^{Y} repräsentiert wird von der Formel ii-a':

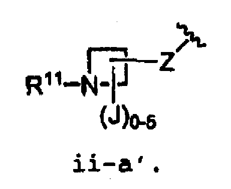

8. Verbindung nach einem der Ansprüche 1 bis 7 wie repräsentiert von Formel Ia:

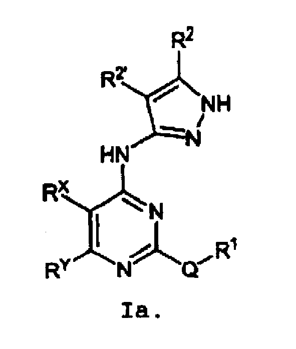

9. Verbindung nach einem der Ansprüche 1 bis 7 wie repräsentiert von Formel Ib:

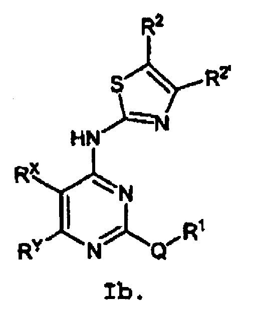

10. Verbindung nach Anspruch 8 oder 9, wobei R^{2'} H oder wahlweise substituiertes C₁- bis C₃-aliphatisch bedeutet.

11. Verbindung nach einem der Ansprüche 8 bis 10, wobei R² H oder wahlweise substituiertes C₁- bis C₃-aliphatisch bedeutet.

12. Verbindung nach einem der Ansprüche 8 bis 10, wobei R⁵ eine wahlweise substituierte Gruppe bedeutet, die aus C₁- bis C₆-aliphatisch oder Phenyl ausgewählt ist.

13. Die folgenden Verbindungen:

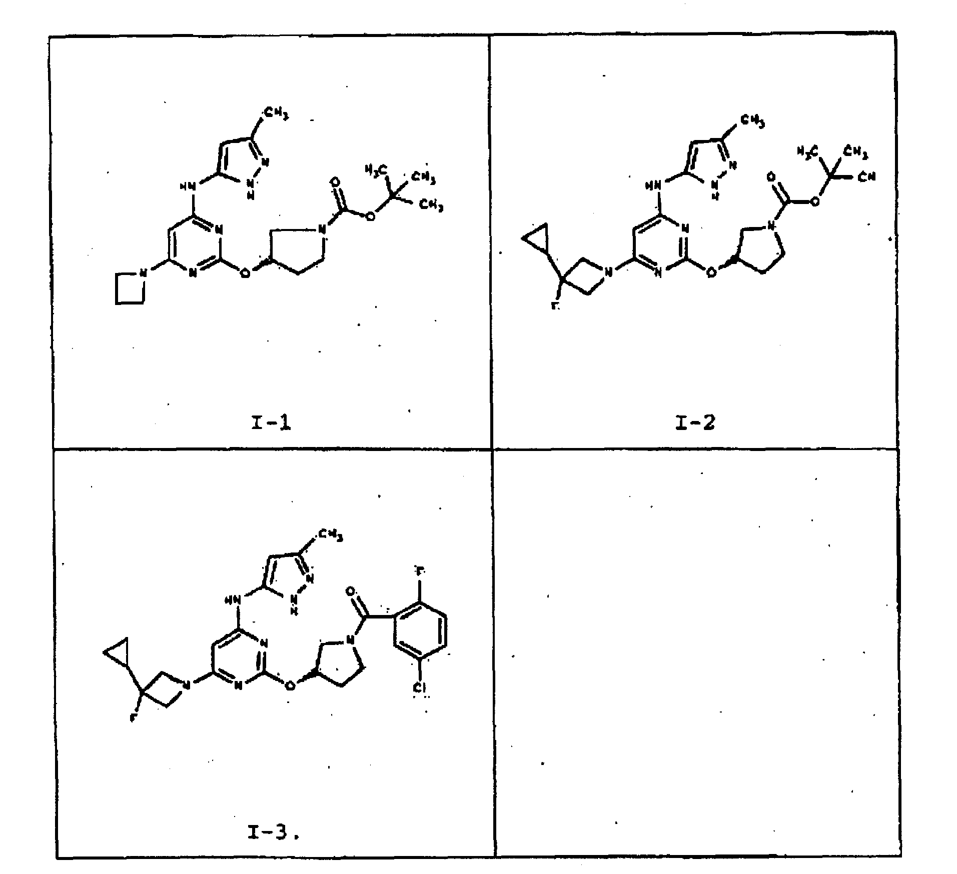

14. Zusammensetzung, die eine Verbindung nach einem der Ansprüche 1 bis 13 und einen Träger, ein Adjuvans oder ein Vehikel, der/das pharmazeutisch verträglich ist, umfasst.

15. Verfahren zum Inhibieren der Aurora-Protein-Kinase-Aktivität in einer biologischen Probe, welches das In-Berührung-Bringen der biologischen Probe mit einer Verbindung nach einem der Ansprüche 1 bis 13 umfasst.

16. Verwendung einer Verbindung nach einem der Ansprüche 1 bis 13 zur Herstellung eines Arzneimittels für die Behandlung einer proliferativen Störung bei einem Patienten, insbesondere einer proliferativen Störung, die aus Melanomen, Myelomen, Leukämien, Lymphomen, Neuroblastomen oder einem Krebs ausgewählt ist, der aus Darm-, Brust-, Magen-, Eierstock-, Gebärmutterhals-, Lungen-, Zentralnervensystem-(ZNS-), Nieren-, Prostata-, Blasen-, Bauchspeicheldrüsen-, Gehirn-(Gliom-), Kopf- und Hals-, Nieren-, Leber-, Melanom-, Sarkom- oder Schilddrüsenkrebs ausgewählt ist.

17. Verwendung einer Verbindung nach einem der Ansprüche 1 bis 13 oder eines pharmazeutisch verträglichen Salzes davon und eines weiteren therapeutischen Mittels, insbesondere von Taxanen, Inhibitoren von BCR-ABL, Inhibitoren von EGFR, DNA-schädigenden Mitteln und/oder Antimetaboliten oder Paclitaxel, Gleevec, Dasatinib, Nilotinib, Tarceva, Iressa, Cisplatin, Oxaliplatin, Kohlenstoffplatin, Anthracyclinen, AraC und/oder 5-FU und/oder Camptothecin, Doxorubicin, Idarubicin, Cisplatin, Taxol, *Taxotère*, Vincristin, Tarceva, MEK-Inhibitor, U0126, KSP-Inhibitor, Vorinostat, Gleevec, Dasatinib und/oder Nilotinib, zur Herstellung eines Arzneimittels für die Behandlung von Krebs bei einem Lebewesen, das ihrer bedarf.

## Revendications

1. Composé de formule I: 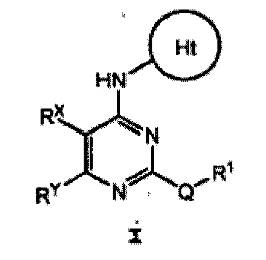 ou un sel pharmaceutiquement acceptable de celui-ci, dans laquelle:
Ht est une thiazole ou une pyrazole; Ht est le cas échéant, et independamment, substitué par R² et R2'; Ht est en particulier 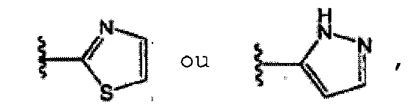 où Ht est le cas échéant et indépendamment substitué par R² et R^{2'} ;
Q représente -O-, -NR'-, -S-, -C(R')₂- ou -(C=O)-;
R^{x} représente H, aliphatique en C₁₋₆, NO₂, CN, halo, NH₂, N(aliphatique en C₁₋₄), N (aliphatique en C₁₋₄)_{2,} O (aliphatique en C₁₋₄), OH, ou -N (C=O) (aliphatique en C₁₋₄) ; où ledit aliphatique est le cas échéant substitué par 1-3 fluoro;
R^{y} représente -Z-R¹⁰;
R¹ représente T-(cycle D);
cyle D est un hétérocyclyle monocyclique à 4-7 chaînons ou un cycle carbocyclyle, dans lequel ledit hétérocyclyle possède 1-4 hétéroatomes de cycle, choisis parmi O, N et S; cycle D est le cas échéant fusionné à un cycle D';
cycle D' est un cycle monocyclique à 5-8 chaînons, partiellement saturé ou complètement insaturé, contenant 0-4 hétéroatomes de cycle, choisis parmi azote, oxygène ou souffre;
cycle D et cyle D' sont le cas échéant chacun, et indépendamment, substitués par 0-4 occurrences de oxo ou -Z-R⁵;
chaque T représente indépendamment une chaîne alkylidène en C₁₋₄ ou est absent;
R² et R^{2'} représentent indépendamment -R, -WR⁶, ou R⁸ ; ou R² et R^{2'} sont pris ensemble avec leurs atomes intermédiaires pour former un cycle fusionné à 5-8 chaînons, insaturé ou partiellement insaturé, possédant 0-3 hétéroatomes de cycle choisis parmi azote, oxygène ou soufre; le cycle à 5-8 chaînons est substitué indépendamment par halo, oxo, -CN, -NO₂, -R⁷ ou -Y-R⁶;
chaque Z, Y et W représente indépendamment une liaison ou une chaîne alkylidène en C₁₋₁₀ dans laquelle jusqu'à six unités méthylène de la chaîne alkylidène sont le cas échéant remplacées par V;
chaque V est choisi parmi -O-, -C(=O)-, -S(O)-, -S(O)₂-, -S- ou -N(R⁴)-;
chaque R³ et R⁵ représente indépendamment -R, -halo, -OR, -C (=O) R, , -CO₂R, -COCOR, COCH₂COR, -NO₂, -CN, -S(O)R, -S(O)₂R, -SR, -N(R⁴)₂, -CON(R⁷)₂, -SO₂N(R⁷)₂, -OC(=O)R, -N(R⁷)COR, -N(R⁷)CO₂ (aliphatique en C₁₋₆), -N(R⁴)N(R⁴)₂, -C=NN(R⁴)₂, -C=N-OR, -N(R⁷)CON(R⁷)₂, -N(R⁷)SO₂N (R⁷) ₂, -N (R⁴) SO₂R, ou -OC(=O) N (R⁷)₂;
chaque R représente l'hydrogène, un groupement aliphatique en C₁₋₆, un cycle aryle en C₆₋₁₀, un cycle hétéroaryle comptant 5-10 atomes de cycle, ou un cycle hétérocyclyle comptant 4-10 atomes de cycle; ledit cycle hétéroaryle ou hétérocyclyle possède 1-4 atomes de cycle choisis parmi azote, oxygène ou souffre; R est le cas échéant substitué par 0-6 R⁹;
chaque R⁴ représente -R⁷, -COR⁷, -CO₂R⁷, -CON(R⁷)₂ ou -SO₂R⁷;
chaque R⁶ représente indépendamment l'hydrogène ou un groupement aliphatique en C₁₋₆ le cas échéant substitué ; ou deux groupements R⁶ sur le même atome d'azote sont pris ensemble avec l'atome d'azote pour former un cycle hétérocyclyle ou hétéroaryle à 4-6 chaînons, le cas échéant substitué;
chaque R⁷ représente indépendamment l'hydrogène ou un groupement aliphatique en C₁₋₆ le cas échéant substitué; ou deux R⁷ sur le même azote sont pris ensemble avec l'azote pour former un cycle hétérocyclyle ou hétéroaryle à 4-8 chaînons, le cas échéant substitué, contenant 1-4 hétéroatomes choisis parmi azote, oxygène ou soufre;
chaque R⁸ représente halogène, -CN ou -NO₂;
chaque R⁹ représente -R', -halo, -OR', -C(=O)R', -CO₂R', -COCOR', COCH₂COR', -NO₂, -CN, -S(O)R', -S(O)₂R', -SR', -N(R')₂, -CON(R')₂, -SO₂N(R')₂, -OC(=O)R', -N(R')COR' , -N(R')CO₂ (aliphatique en C₁₋₆), -N (R') N (R') ₂, -N (R') CON (R (') ₂, -N (R') SO₂N (R') ₂, -N (R') SO₂R', -OC (=O) N (R') ₂, =NN (R') ₂, =N-OR', ou =O;
chaque R¹⁰ représente un cycle hétérocyclique à 4 chaînons contenant 1 hétéroatome choisi parmi O, NR¹¹, et S; chaque R¹⁰ est le cas échéant substitué par 0-6 occurrences of J;
chaque J représente indépendamment R, -halo, -OR, oxo, -C (=O) R, -CO₂R, -COCOR, -COCH₂COR, -NO₂, -CN, -S (O) R, -S (O) ₂R, -SR, -N (R⁴)₂, -CON (R⁷) ₂, -SO₂N (R⁷) ₂, -C (=O) R, -N (R⁷) COR, -N (R⁷) CO₂ (aliphatique en C₁₋₆), -N (R⁴) N (R⁴) ₂, =NN (R⁴) ₂, =N-OR, -N(R⁷)CON(R⁷)₂, -N(R⁷)SO₂N(R⁷)₂, -N(R⁴)SO₂R, -OC(=O)N(R⁷)₂, ou -OP(=O)(OR")₂; ou
2 groupements J, sur le même atome ou des atomes différents, forment avec le ou les atomes auxquels ils sont liés, un cycle saturé, partiellement saturé ou insaturé, à 3-8 chaînons, comptant 0-2 hétéroatomes choisis parmi O, N, or S; 1-4 atomes d'hydrogène sur le cycle formé par les 2 groupements J sont le cas échéant remplacés par halo, alkyle en C₁₋₃, ou -O(alkyle en C₁₋₃) ; ou deux atomes d'hydrogène sur le cycle sont le cas échéant remplacés par oxo, ou par un spiro-cycloalkyle en C₃₋₄; ledit alkyle en C₁₋₃ est le cas échéant substitué par 1-3 fluor;
chaque R¹¹ représente -R⁷, -COR⁷, -CO₂R⁷, -CON(R⁷)₂, ou -SO₂R⁷;
chaque R' représente indépendamment l'hydrogène ou un groupement aliphatique en C₁₋₆, le cas échéant substitué par 0-4 occurrences de NH₂, NH(aliphatique en C₁₋₄), N(aliphatique en C₁₋₄)₂, halogène, aliphatique en C₁₋₄, OH, O (aliphatique en C₁₋₄), NO₂, CN, CO₂H, CO₂ (aliphatique en C₁₋₄), O (halo aliphatique en C₁₋₄), ou halo aliphatique en C₁₋₄; ou deux R' ensemble avec le ou les atomes auxquels ils sont attachés, forment un carbocyclyle ou un hétérocyclyle le cas échéant substitué, à 3-6 chaînons; and chaque R" représente indépendamment H ou un alkyle en C₁₋₂,
dans laquelle un groupement aryle (incluant aralkyle, aralkoxy, aryloxyalkyle) ou hétéroalkyle (incluant hétéroaralkyl et hétéroarylalkoxy) peut contenir un ou plusieurs substituants et ainsi être "éventuellement substitué";
dans laquelle sauf défini autrement ci-dessus et ici, les substituants qui conviennent sur l'atome de carbone insaturé d'un groupement aryle ou hétéroaryle sont choisis parmi un halogène; -R^{o}; -OR^{o}; -SR^{o}; phényle (Ph) éventuellement subtitué avec R^{o}; -O(Ph) éventuellement substitué avec R^{o}; -(CH₂)₁₋₂(Ph); éventuellement substitué avec -R^{o}; -CH=CH(Ph), éventuellement susbtitué avec R^{o}; un cycle hétéroaryle ou hétérocyclique à 4-6 chaînons éventuellement substitué par R^{o}; -NO₂; -CN; -N(R^{o})₂; -NR^{o}C(O)R^{o}; -NR^{o}C(S)R^{o}; -NR^{o}C(O)N(R^{o})₂; -NR^{o}C(S)N(R^{o})₂;
-NR^{o}CO₂R^{o}; -NR^{o}NR^{o}-, NR^{o}NR^{o}C(O)R^{o}; NR^{o}NR^{o}C(O)N(R^{o})₂; NR^{o}NR^{o}CO₂R^{o}; -C(O)C(O)R^{o}; -C(O)CH₂C(O)R^{o}; -CO₂R^{o}; -C(O)R^{o}; C(S)R^{o}; -C(O)N(R^{o})₂; -C(S)N(R^{o})₂; -OC(O)N(R^{o})₂; -OC(O)R^{o}; -C(O)N(OR^{o})R^{o}; -C(NOR^{o})R^{o}; -S(O)₂R^{o}; -S(O)₃R^{o}; -SO₂N(R^{o})₂; -S(O)R^{o}; -NR^{o}SO₂N(R^{o})₂; -NR^{o}SO₂R^{o}; -N(OR^{o})R^{o}; -C(=NH)-N(R^{o})₂; C(=NH)-OR^{o}; -P(O)₂R^{o}; -PO(R^{o})₂; -OPO(R^{o})₂; ou -(CH₂)₀₋₂NHC(O)R^{o}; dans lequel chaque apparition indépendante de R^{o} est choisi parmi l'hydrogène, un aliphatique en C1-6 éventuellement substitué; un cycle hétéroaryle ou un hétérocyclyle non substitué à 4-6 chaînons, phényle, -O(Ph), ou -CH₂(Ph), ou, nonobstant la définition ci-dessous, deux apparitions indépendantes de R^{o}, sur le même substituant ou sur des substituants différents, pris ensemble avec l'atome ou les atomes auxquels chaque groupement R^{o} est lié, forment un cycle monocyclique ou bicyclique éventuellement susbtitué à 3-12 chaînons saturés, partiellement insaturés ou entièrement insaturés, ayant 0-4 hétéroatomes indépendamment choisis parmi l'azote, l'oxygène, ou le soufre et
dans laquelle les substituants éventuels sur le groupe aliphatique de R^{o} sont choisis parmi NH2, NH(C₁₋₄aliphatique), N(C₁₋₄aliphatique)₂, halogène, C₁₋₄ aliphatique, OH, O(C₁₋₄aliphatique); NO₂, CN, CO₂H, CO₂ (C₁₋₄aliphatique); O (haloC₁₋₄ aliphatique), ou haloC₁₋₄ aliphatique, dans lequel chacun des groupements de R^{o} ci-avant est non substitué.

2. Composé selon la revendication 1, dans lequel le cycle D est un cycle monocyclique à 4-7 chaînons, le cas échéant substitué, choisi parmi un cycle hétérocyclyle, en particulier un cycle pipéridinyle ou pyrrolidinyle le cas échéant substitué, ou un cycle carbocyclyle, ledit cycle hétérocyclique comptant 1-2 hétéroatomes choisis parmi O, N et S.

3. Composé selon la revendication 1 ou 2, dans lequel R² représente H ou un aliphatique en C₁₋₆.

4. Composé selon l'une quelconque des revendications 1-3, dans lequel Z est absent.

5. Composé selon l'une quelconque des revendications 1-4, dans lequel Z représente une chaîne alkylidène en C₁₋₆, en particulier une chaîne alkylidène en C₁₋₄, dans laquelle 1-2 unités méthylène de Z sont le cas échéant remplacées par O, -N(R⁶)- ou S.

6. Composé selon l'une quelconque des revendications 1-5, dans lequel R¹⁰ est une azétidine, le cas échéant substituée; en particulier, R¹⁰ est représenté par la formule i:

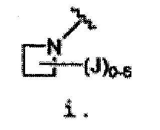

7. Composé selon l'une quelconque des revendications 1-5, dans lequel R^{y} est représenté par la formule ii-a':

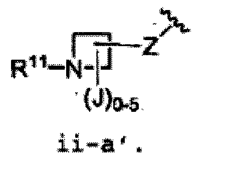

8. Composé selon l'une quelconque des revendications 1-7, représenté par la formule Ia:

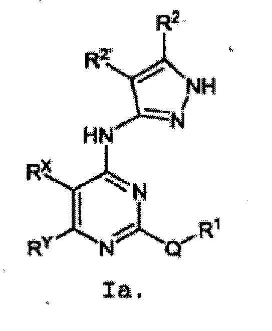

9. Composé selon l'une quelconque des revendications 1-7, représenté par la formule Ib:

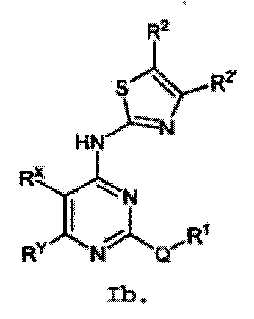

10. Composé selon la revendication 8 ou 9, dans lequel R^{2'} représente H ou un aliphatique en C₁₋₃ le cas échéant substitué.

11. Composé selon l'une quelconque des revendications 8-10, dans lequel R² représente H ou un aliphatique en C₁₋₃ le cas échéant substitué.

12. Composé selon l'une quelconque des revendications 8-10, dans lequel R⁵ est un groupement, le cas échéant substitué, choisi parmi aliphatique en C₁₋₆ ou phényle.

13. Les composés suivants :

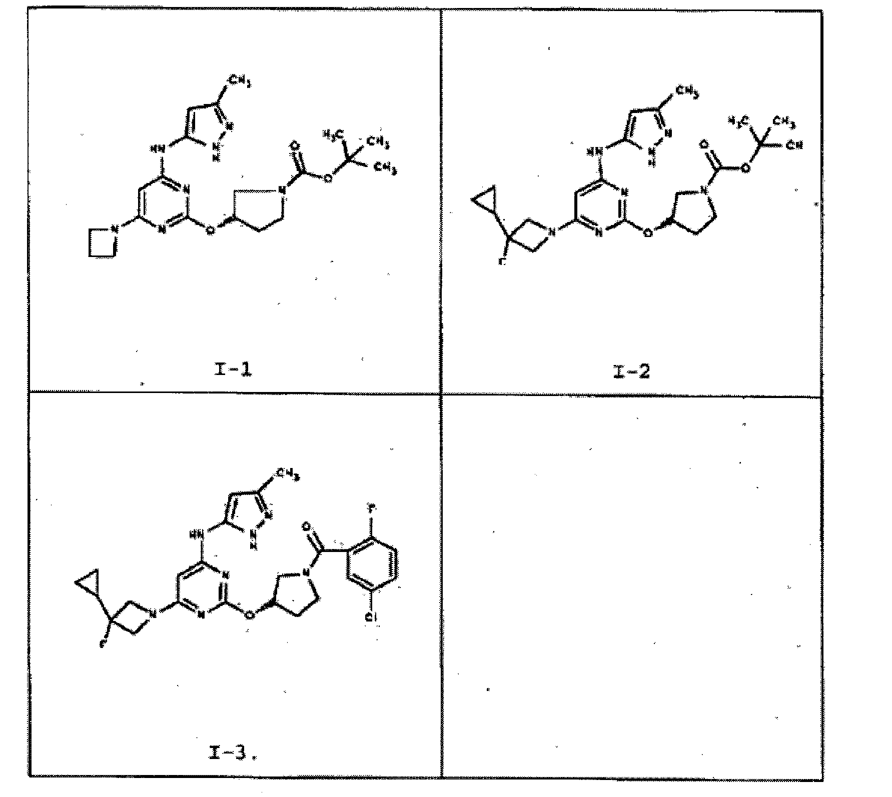

14. Composition comprenant un composé selon l'une quelconque des revendications 1-13, et un support, adjuvant ou véhicule pharmaceutiquement acceptable.

15. Procédé d'inhibition de l'activité de kinase de la protéine Aurora dans un échantillon biologique, comprenant la mise en contact dudit échantillon biologique avec un composé selon l'une quelconque des revendications 1-13.

16. Utilisation d'un composé selon l'une quelconque des revendications 1-13 dans la fabrication d'un médicament pour traiter un trouble prolifératif chez un patient, en particulier un trouble prolifératif choisi parmi mélanome, myélome, leucémie, lymphome, neuroblastome, ou un cancer choisi parmi cancer du côlon, du sein, gastrique, ovarien, cervical, pulmonaire, du système nerveux central (CNS), rénal, de la prostate, de la vessie, du pancréas, du cerveau (gliomes), de la tête et de la nuque, des reins, du foie, mélanome, sarcome ou cancer de la thyroïde.

17. Utilisation d'un composé selon l'une quelconque des revendications 1-13, ou d'un sel pharmaceutiquement acceptable de celui-ci, et un autre agent thérapeutique, en particulier taxanes, inhibiteurs de bcr-abl, inhibiteurs de EGFR, agents destructeurs d'ADN, et/ou antimétabolites ou Paclitaxel, Gleevec, dasatinib, nilotinib, Tarceva, Iressa, cisplatine, oxaliplatine, carboplatine, anthracyclines, AraC et/ou 5-FU et/ou camptothécine, doxorubicine, idarubicine, Cisplatine, taxol, taxotere, vincristine, tarceva, l'inhibiteur de MEK, U0126, un inhibiteur de KSP, vorinostat, Gleevec, dasatinib, et/ou nilotinib, pour la fabrication d'un médicament pour traiter un cancer chez un sujet qui le requiert.
